(19) Europäisches Patentamt
European Patent Office
Office européen des brevets

(11) **EP 4 466 274 B1**

(12) **EUROPEAN PATENT SPECIFICATION**

(45) Date of publication and mention
of the grant of the patent:
**15.07.2026  Bulletin 2026/29**

(21) Application number: **22705507.6**

(22) Date of filing: **20.01.2022**

(51) International Patent Classification (IPC):
*C07F 1/00* $^{(2006.01)}$        *A61P 3/04* $^{(2006.01)}$
*A61P 3/10* $^{(2006.01)}$       *A61P 25/00* $^{(2006.01)}$
*A61P 27/06* $^{(2006.01)}$

(52) Cooperative Patent Classification (CPC):
**C07F 1/005; A61P 3/04; A61P 3/10; A61P 25/00;
A61P 27/06**

(86) International application number:
**PCT/CN2022/073051**

(87) International publication number:
**WO 2023/137672 (27.07.2023 Gazette 2023/30)**

(54) **L-N-ISOBUTYRYL CYSTEINE (L-NIBC) -BOUND GOLD CLUSTER AU 18(L-NIBC) 14 COMPOSITIONS AND APPLICATIONS THEREOF**

L-N-ISOBUTYRYLCYSTEIN-(L-NIBC)-GEBUNDENE GOLDCLUSTER AU 18(L-NIBC) 14-ZUSAMMENSETZUNGEN UND ANWENDUNGEN DAVON

18 AGRÉGAT D'OR LIÉ À L-N-ISOBUTYRYL CYSTÉINE (L-NIBC) AU(L-NIBC) 14 COMPOSITIONS ET UTILISATIONS ASSOCIÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.11.2024  Bulletin 2024/48**

(73) Proprietor: **Shenzhen Profound View
Pharmaceutical
Technology Co., Ltd.
Shenzhen, Guangdong 518000 (CN)**

(72) Inventor: **SUN, Taolei
Wuhan, Hubei 430070 (CN)**

(74) Representative: **Abel & Imray LLP
Westpoint Building
James Street West
Bath BA1 2DA (GB)**

(56) References cited:
**WO-A1-2021/184762     WO-A1-2021/226736**

**Description**

**FIELD OF THE INVENTION**

The present invention relates to gold clusters (AuCs), particularly to L-N-isobutyryl cysteine (L-NIBC)-bound gold cluster molecule $Au_{18}(L\text{-}NIBC)_{14}$, composition comprising the gold cluster molecule $Au_{18}(L\text{-}NIBC)_{14}$, use of the gold cluster molecule $Au_{18}(L\text{-}NIBC)_{14}$ to prepare medications for treatment of various diseases.

**BACKGROUND OF THE INVENTION**

Gold clusters (AuCs) refer to one kind of substances that contains ligand-bound gold core and the diameter of the gold core is less than 3 nm. Due to energy level splitting, gold clusters show molecule-like characteristics, so each of gold cluster molecules in gold clusters can be expressed by a molecular formula. However, in gold clusters, the number of gold atoms of gold cluster molecules can vary from 2 to 1000, and the number of ligands can also vary from 1 to 1000; what makes the situation more complicated is that even for gold clusters with the same number of gold atoms, the number of ligands varies with ligand types and preparation conditions. Therefore, as for the gold cluster molecules with specific number of gold atoms and specific number of ligands that might are present in the same ligand-bound gold clusters, it is still unknown. Gold clusters used to treat cirrhosis are disclosed in WO 2021/184762 A1 and WO 2021/226736 A1.

**SUMMARY OF THE INVENTION**

The present invention provides an L-NIBC-bound gold cluster molecule $Au_{18}(L\text{-}NIBC)_{14}$, the L-NIBC-bound gold cluster molecule $Au_{18}(L\text{-}NIBC)_{14}$ for use of treatment of diseases in a subject, a composition comprising the L-NIBC-bound gold cluster molecule $Au_{18}(L\text{-}NIBC)_{14}$, and a compound for use in a method of treating diseases in a subject with the L-NIBC-bound gold cluster molecule $Au_{18}(L\text{-}NIBC)_{14}$.

In certain embodiments, the present invention provides an L-N-isobutyryl cysteine (L-NIBC)-bound gold cluster molecule comprising a gold core; and a ligand L-NIBC bound to the gold core; wherein the L-NIBC-bound gold cluster molecule consisting of 18 gold atoms, and 14 L-NIBC ligands which has a formula of $Au_{18}(L\text{-}NIBC)_{14}$.

In certain embodiments, the present invention provides the L-NIBC-bound gold cluster molecule $Au_{18}(L\text{-}NIBC)_{14}$ for use of manufacture of medicament for treatment of a disease in a subject. In certain embodiments, the disease includes multiple sclerosis, Alzheimer's disease (AD), liver cirrhosis, diabetes, Parkinson's disease (PD), and glaucoma.

In certain embodiments, the present invention provides a composition for treatment of a disease in a subject, wherein the composition comprises the L-NIBC-bound gold cluster molecule $Au_{18}(L\text{-}NIBC)_{14}$. In certain embodiments, when the L-NIBC-bound gold cluster molecule $Au_{18}(L\text{-}NIBC)_{14}$ is used as a component in a mixture of gold cluster molecules in the composition, the L-NIBC-bound gold cluster molecule $Au_{18}(L\text{-}NIBC)_{14}$ has a chromatographic content of at least 7% in the mixture. In certain embodiments, the disease includes multiple sclerosis, Alzheimer's disease (AD), liver cirrhosis, diabetes, Parkinson's disease (PD), and glaucoma.

In certain embodiments, the present invention provides a compound for use in a a method for treatment of a disease in a subject, said method comprising administering a composition to the subject, wherein the composition comprises the L-NIBC-bound gold cluster molecule $Au_{18}(L\text{-}NIBC)_{14}$; and wherein the disease is one selected from the group consisting of multiple sclerosis, Alzheimer's disease (AD), liver cirrhosis, Type 2 diabetes, Parkinson's disease (PD), and glaucoma.

The objectives and advantages of the invention will become apparent from the following detailed description of preferred embodiments thereof in connection with the accompanying drawings.

**Description of the Drawings**

Preferred embodiments according to the present invention will now be described with reference to the Figures, in which like reference numerals denote like elements.

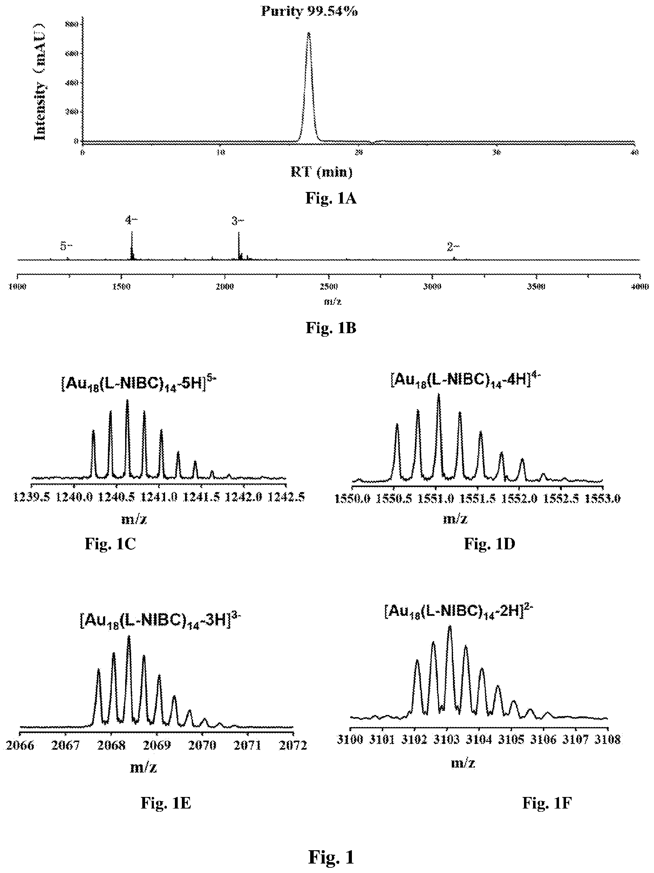

Fig. 1 shows the size exclusion chromatography (SEC) and mass spectra of S8 $(Au_{18}(L\text{-}NIBC)_{14})$; Fig. 1A, the SEC of S8 $(Au_{18}(L\text{-}NIBC)_{14})$; Fig. 1B, ESI mass spectrum of S8 $(Au_{18}(L\text{-}NIBC)_{14})$ (large range); Figs. 1C - 1F, mass spectra isotope peaks displayed after amplification of mass spectrum peaks with different mass charge ratio (M/z), where, in Fig. 1C, z = - 2; in Fig. 1D, z=-3; in Fig. 1E, z=-4; in Fig. 1F, z=-5.

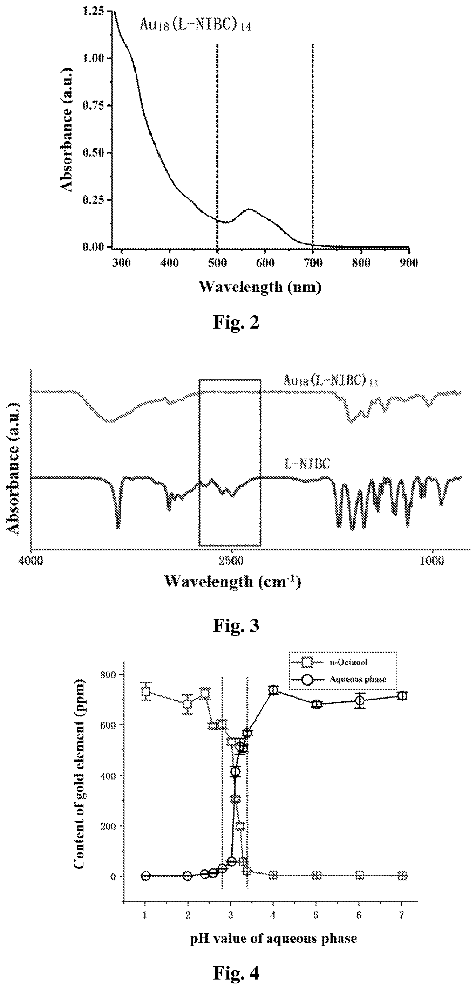

Fig. 2 shows a typical UV-Vis absorption spectrum of S8 $(Au_{18}(L\text{-}NIBC)_{14})$.

Fig. 3 shows infrared absorption spectra of S8 $(Au_{18}(L\text{-}NIBC)_{14})$ (Upper spectrum) and ligand L-NIBC (Lower spectrum).

Fig. 4 shows the changes of distribution of S8 drug in aqueous phase and oil phase along with the changes of the pH values of the initial aqueous solution.

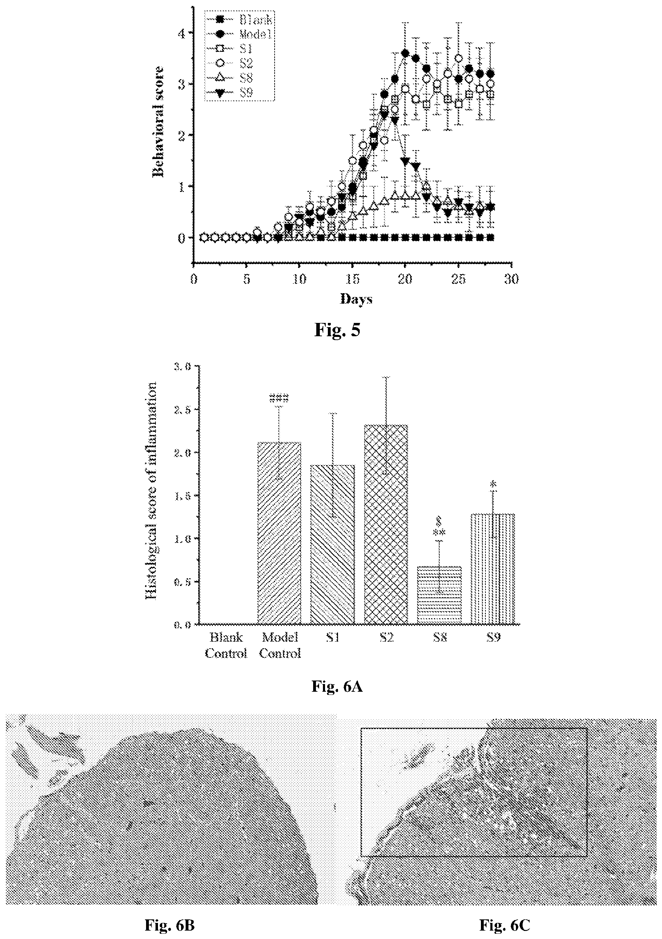

Fig. 5 shows the behavioral clinical scores of EAE model drug test.

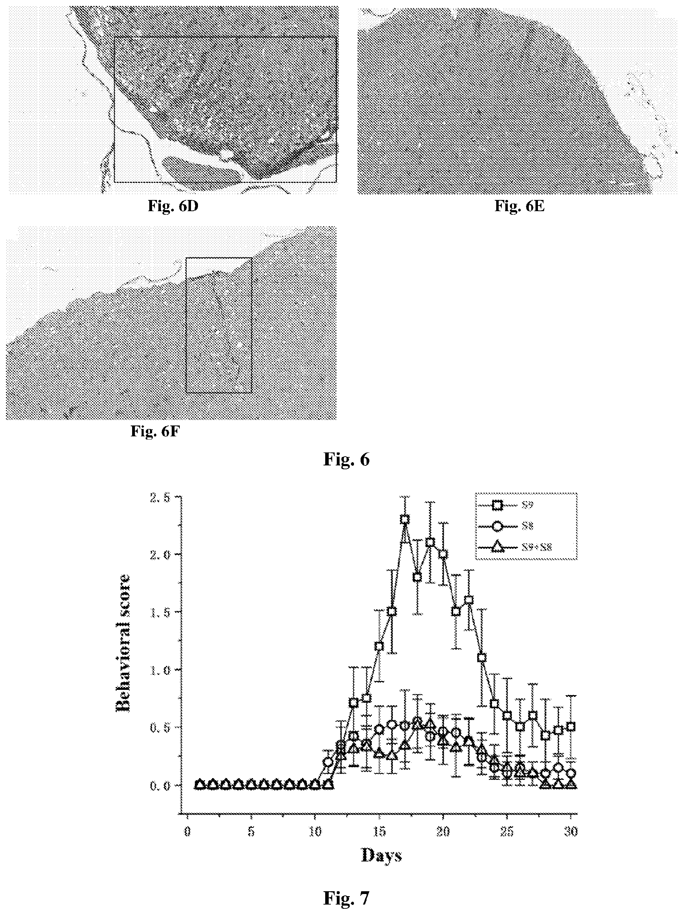

Fig. 6 shows the results of HE staining experiment in EAE mouse model; Fig. 6A, the statistical results of histological score of exemplary drug administration groups, where ###: Compared with blank control group, $P < 0.001$; *: Compared with model control group, $P < 0.05$; **: Compared with model control group, $P < 0.01$; Fig. 6B-6F, respective exemplary images of blank control group, model control group, S1 drug administration group, S8 drug administration group, and S9 drug administration group.

Fig. 7 shows the behavioral clinical scores of S8 drug, S9 drug, and S9+S8 drug in EAE model drug test.

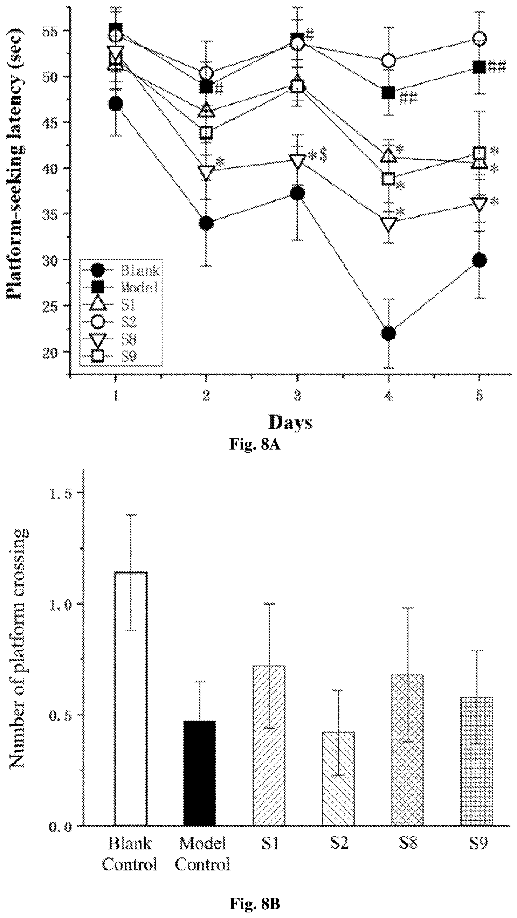

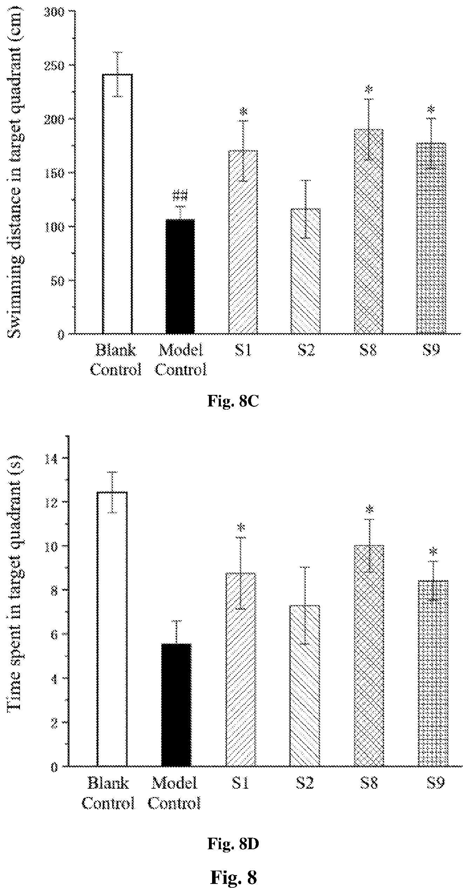

Fig. 8 shows the effects of 180-days consecutive drug administrations of S1, S2, S8, and S9 on the performances of male mice in Morris water maze test; Fig. 8A, platform-seeking latencies during the training of positioning navigation experiment; Fig. 8B, number of platform crossing; Fig. 8C, swimming distance in target quadrant; Fig. 8D, time spent in target quadrant; where, #: Compared with normal blank control group, $P < 0.05$; ##: Compared with normal blank control group, $P < 0.01$; *: Compared with model control group, $P < 0.05$; **: Compared with model control group, $P < 0.01$.

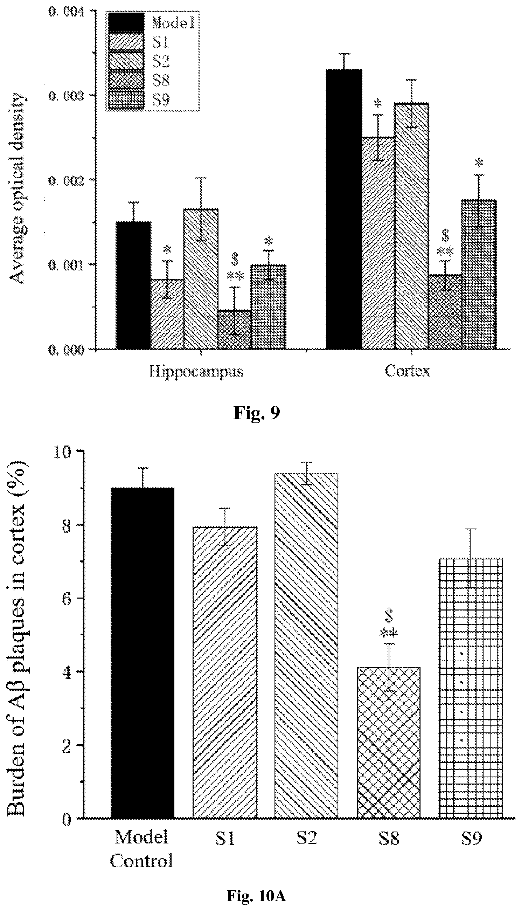

Fig. 9 shows the effects of different gold clusters drugs on the formation of Aβ1-40 plaques in the hippocampus and cortex of APP/PS1 double transgenic AD model mice; where *: Compared with model control group, $P < 0.05$; **: Compared with model control group, $P < 0.01$; $: Compared with S9 drug administration group, $P < 0.05$.

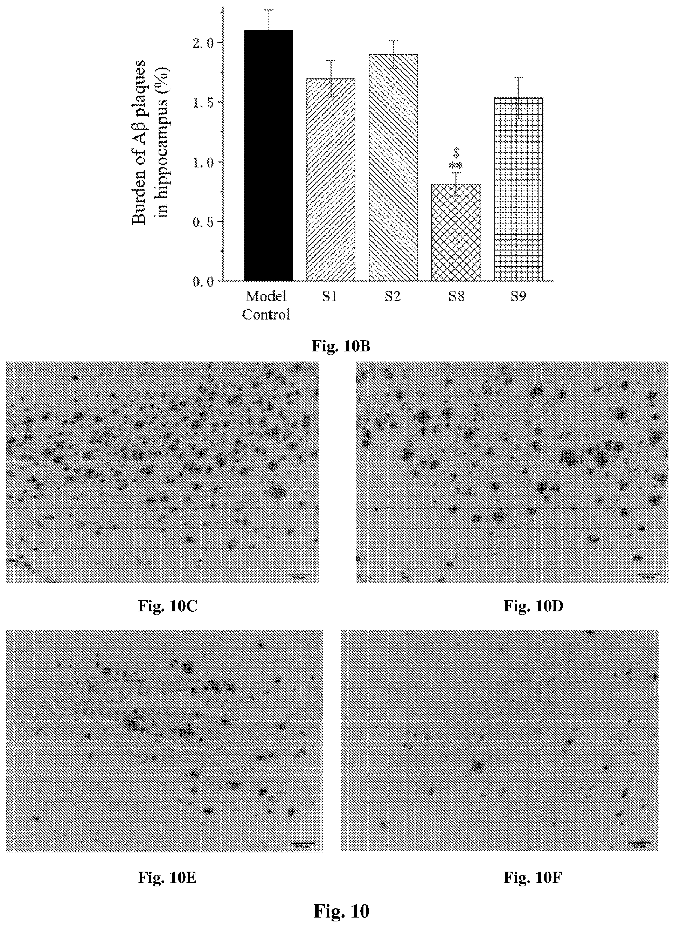

Fig. 10 shows the effects of drugs on the formation of intracerebral Aβ plaques in 5xFAD transgenic mouse model; Fig. 10A, Aβ plaque numbers in cerebral cortex; Fig. 10B, Aβ plaque numbers in hippocampal region; Fig. 10C, an exemplary image of Aβ plaques in the cerebral cortex of mice in model control group; Fig. 10D, an exemplary image of Aβ plaques in the cerebral cortex of mice in S8 drug administration group; Fig. 10E, an exemplary image of Aβ plaques in the hippocampus of mice in model control group; Fig. 10F, an exemplary image of Aβ plaques in the hippocampus of mice in S8 drug administration group; where, **: Compared with model control group, $P < 0.01$; $: Compared with S9 drug administration group, $P < 0.05$.

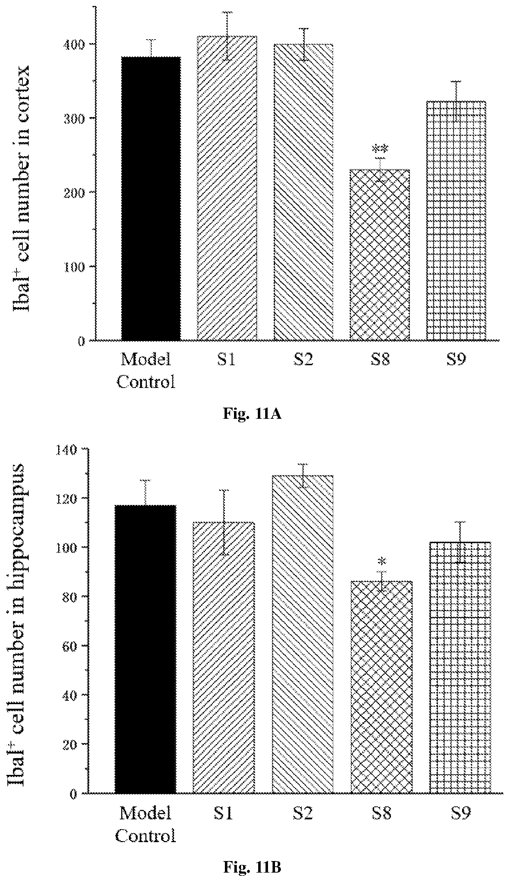

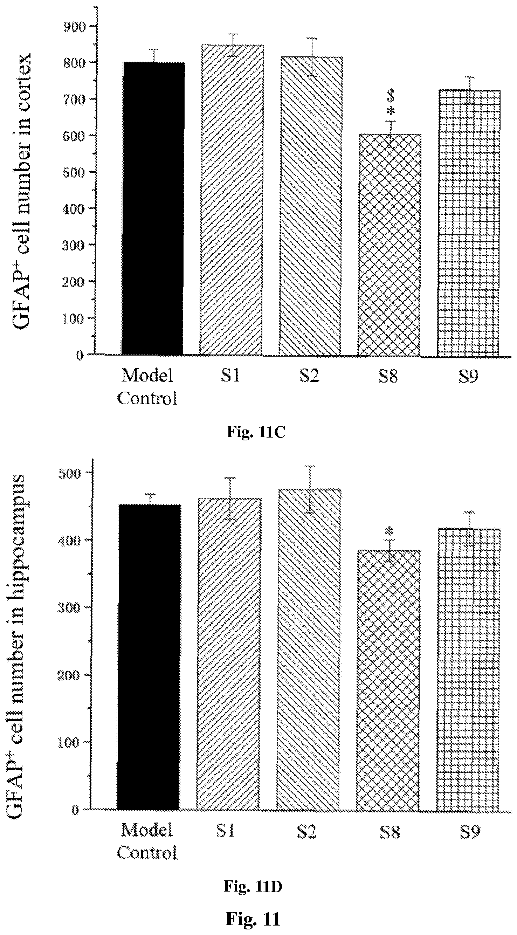

Fig. 11 shows the effects of drugs on the numbers of Iba1+ positive cells and GFAP+ cells in the brain cortex and hippocampus regions of 5xFAD transgenic AD model mice; Fig. 11A, the numbers of Iba1+ positive cells in brain cortex; Fig. 11B, the numbers of Iba1+ positive cells in hippocampus; Fig. 11C, the numbers of GFAP+ positive cells in brain cortex; Fig. 11D, the numbers of GFAP+ positive cells in hippocampus; where *: Compared with model control group, $P < 0.05$; **: Compared with model control group, $P < 0.01$; $: Compared with S9, $P < 0.05$.

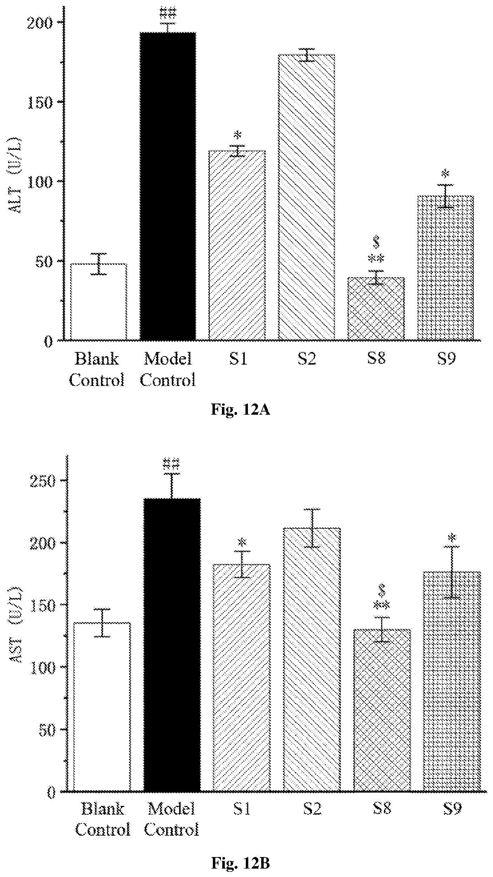

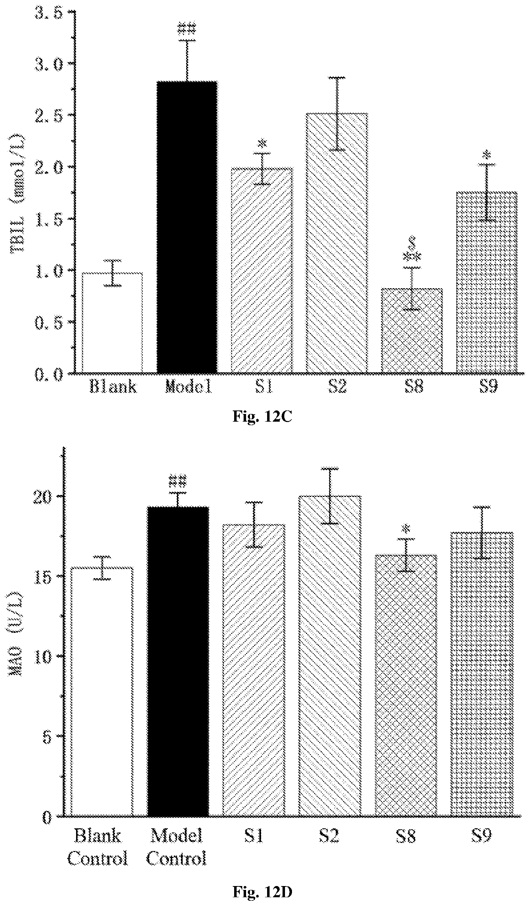

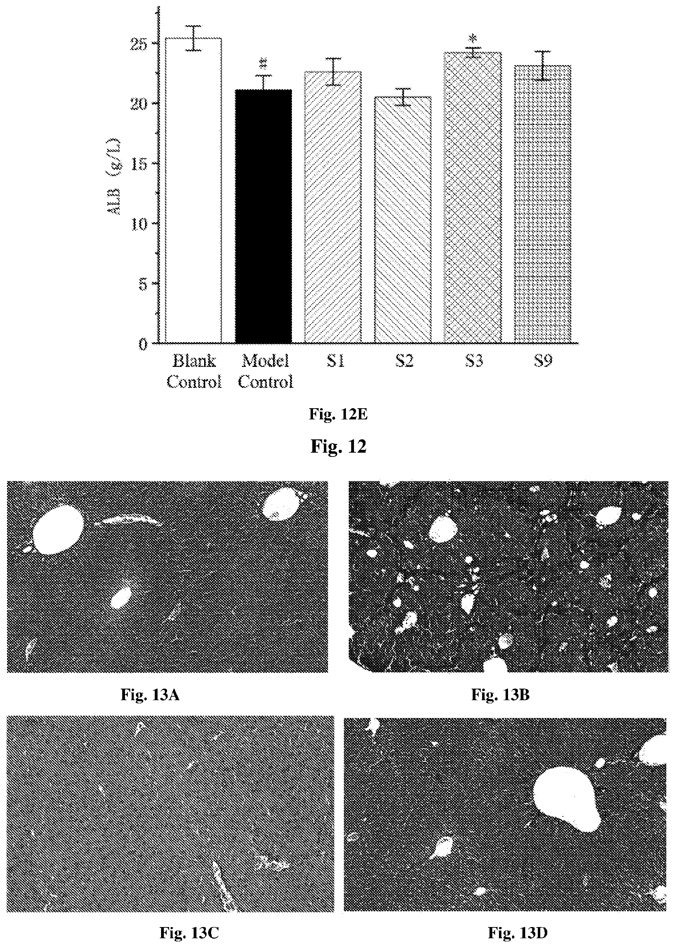

Fig. 12 shows the effects of drugs on serum indexes in $CCl_4$ modeling liver cirrhosis model mice; Fig. 12A, serum levels of ALT; Fig. 12B, serum levels of AST; Fig. 12C, serum levels of TBIL; Fig. 12D, serum levels of MAO; Fig. 12E, serum levels of ALB; where #: Compared with blank control group, $P < 0.05$; ##: Compared with blank control group, $P < 0.01$; *: Compared with model control group, $P < 0.05$; **: Compared with model control group, $P < 0.01$; $: Compared with S9 drug administration group, $P < 0.05$.

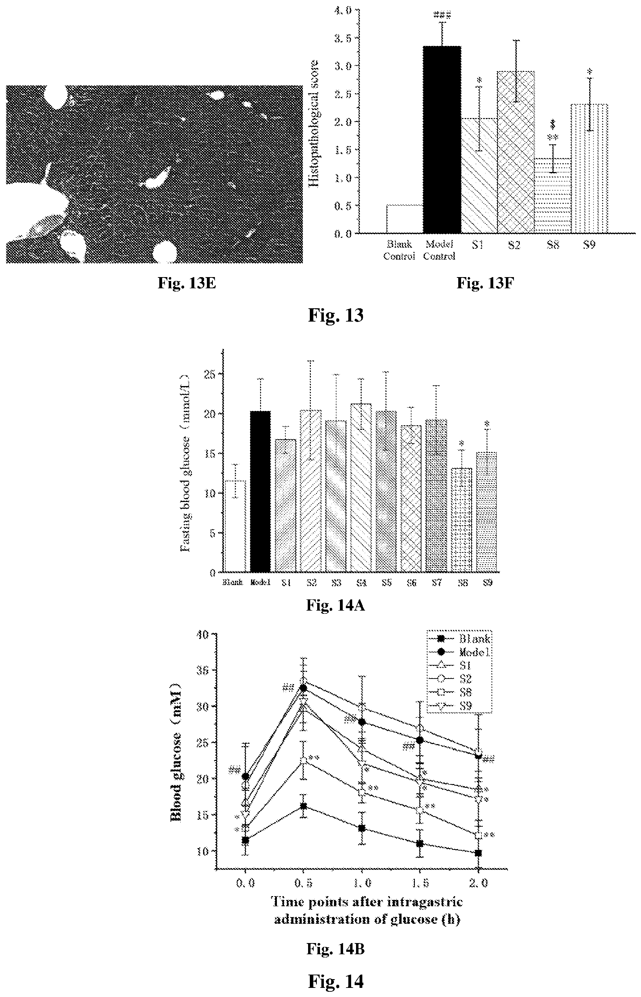

Fig. 13 shows the exemplary HE staining images of liver tissues of $CCl_4$ modeling liver cirrhosis model mice administered with different drugs and pathological scores; Fig. 13A, blank control group; Fig. 13B, model control group; Fig. 13C, S1 drug administration group; Fig. 13D, S8 drug administration group; Fig. 13E, S9 drug administration group; Fig. 13F, pathological scores; where, ###: Compared with blank control group, $P < 0.001$; *: Compared with model control group, $P < 0.05$; **: Compared with model control group, $P < 0.01$; $: Compared with S9 drug administration group, $P < 0.05$.

Fig. 14 shows the results of fasting blood glucose and glucose tolerance levels from Protocol I with simultaneous modeling and drug administration in mouse diabetes model; Fig. 14A, fasting blood glucose levels; Fig. 14B, glucose tolerance levels at different time points after intragastric administration of glucose; where, ##: Compared with blank control group, $P < 0.01$; *: Compared with model control group, $P < 0.05$; **: Compared with model control group, $P < 0.01$.

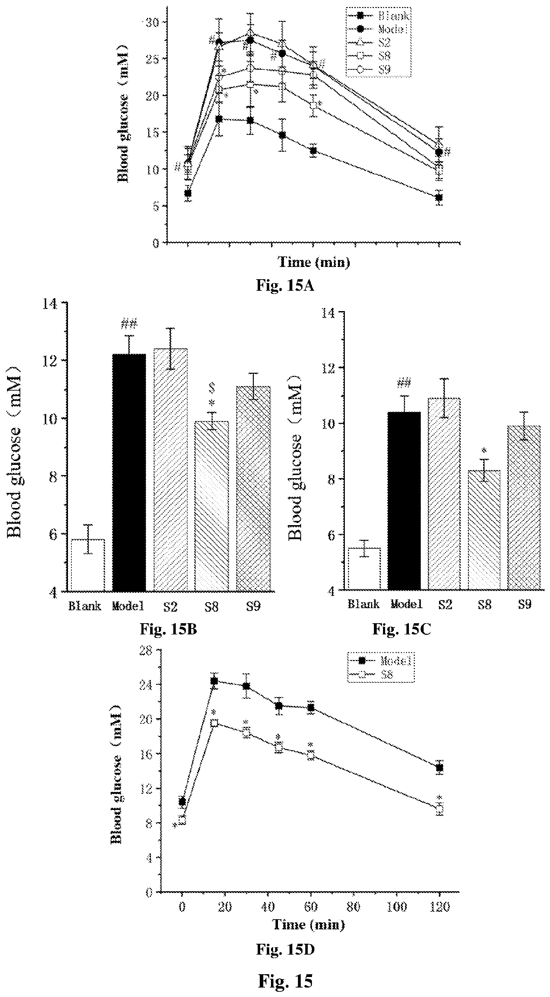

Fig. 15 shows the results of fasting blood glucose and glucose tolerance from Protocol II with drug being differently administered after modeling; Fig. 15A, results of glucose tolerance after acute drug administration; Fig. 15B, results of fasting blood glucose after one-month consecutive drug administration; Fig. 15C, results of fasting blood glucose after two-month consecutive drug administration; Fig. 15D, results of glucose tolerance after two-month consecutive drug administration; where #: Compared with blank control group, $P < 0.05$; ##: Compared with blank control group, $P < 0.01$; *: Compared with model control group, $P < 0.05$; $: Compared S9 drug administration group, $P < 0.05$.

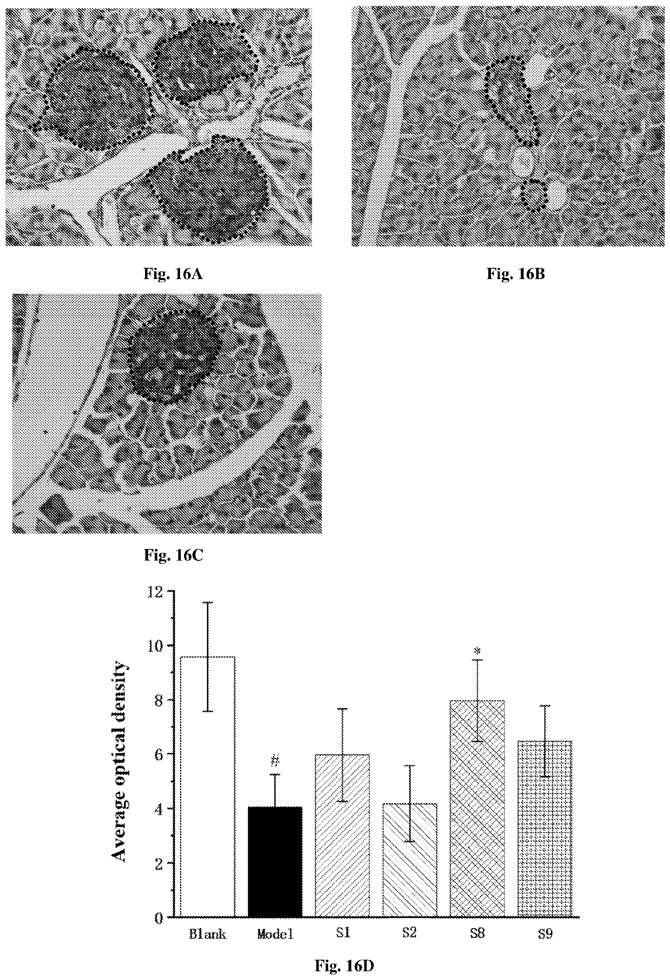

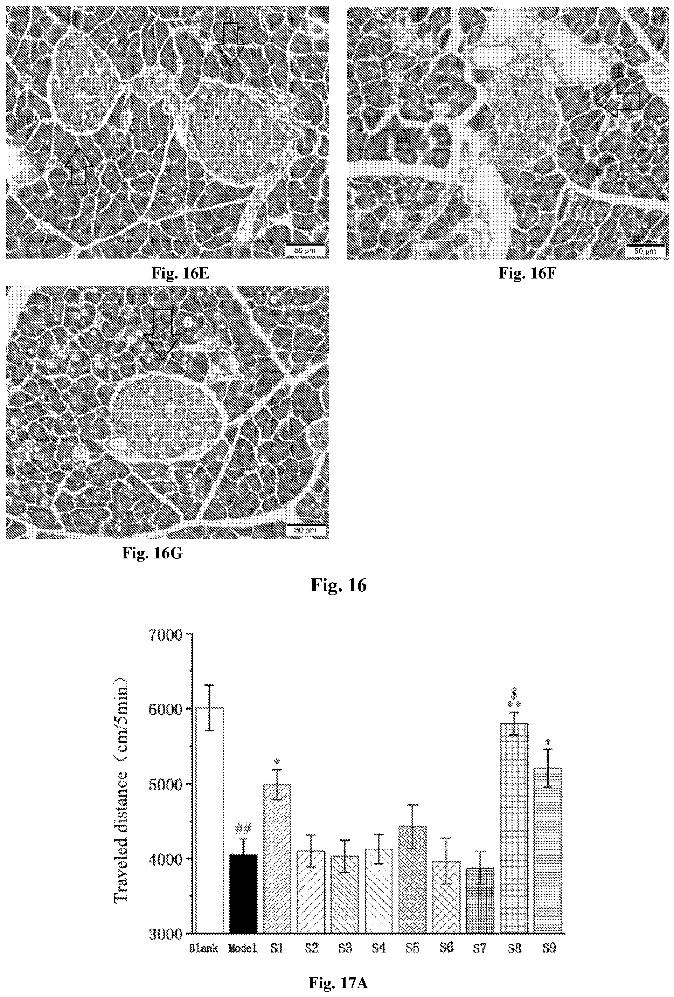

Fig. 16 shows the results of insulin immunohistochemistry from Protocol I with simultaneous modeling and drug administration; Fig. 16A, exemplary image of blank control group; Fig. 16B, exemplary image of model control group; Fig. 16C, exemplary image of S8 drug administration group; Fig. 16D, average optical densities, where #: Compared with blank control group, $P < 0.05$; *: Compared with model control group, $P < 0.05$; Fig. 16E, exemplary image of HE staining of blank control group; Fig. 16F, exemplary image of HE staining of model control group; Fig. 16G, exemplary image of HE staining of S2 drug administration group.

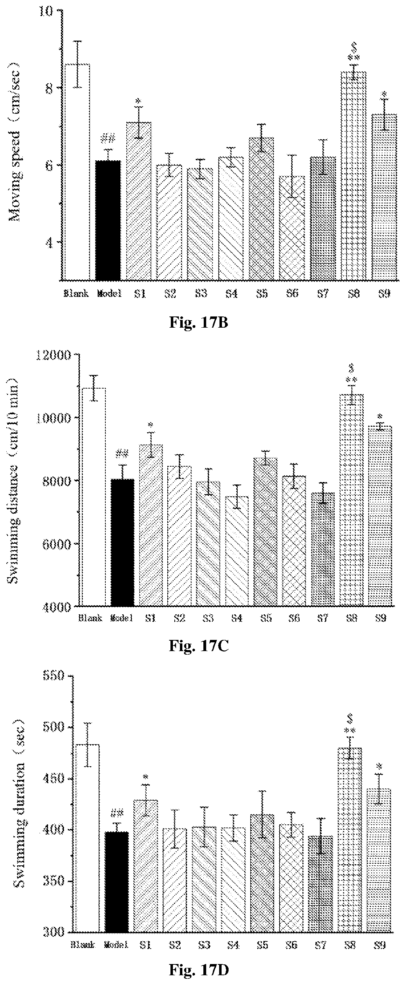

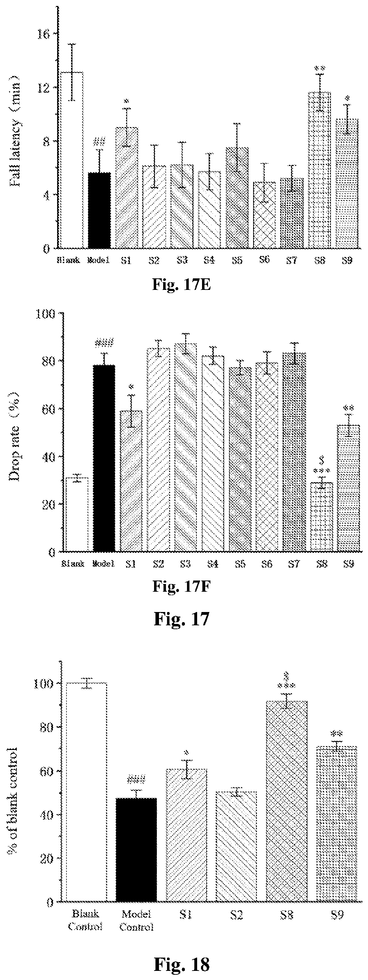

Fig. 17 shows the results of effects of drugs on behavior of MPTP injury-induced PD model mice; Fig. 17A, motion distance; Fig. 17B, moving speed; Fig. 17C, swimming distance; Fig. 17D, swimming duration; Fig. 17E, fall latency; Fig. 17F, drop rate; where, #: Compared with blank control group, $P < 0.05$; ##: Compared with blank control group, $P < 0.01$; ###: Compared with blank control group, $P < 0.001$; *: Compared with model control group, $P < 0.05$; **: Compared with model control group, $P < 0.01$; ***: Compared with model control group, $P < 0.001$; $: Compared with S9 drug administration group, $P < 0.05$.

Fig. 18 shows WB test results of effects of drugs on TH expression in mouse intracerebral striatum in MPTP injury-induced PD model; where, ##: Compared with blank control group, $P < 0.01$; *: Compared with model control group, $P < 0.05$; **: Compared with model control group, $P < 0.01$; ***: Compared with model control group, $P < 0.001$; $: Compared with S9 drug administration group, $P < 0.05$.

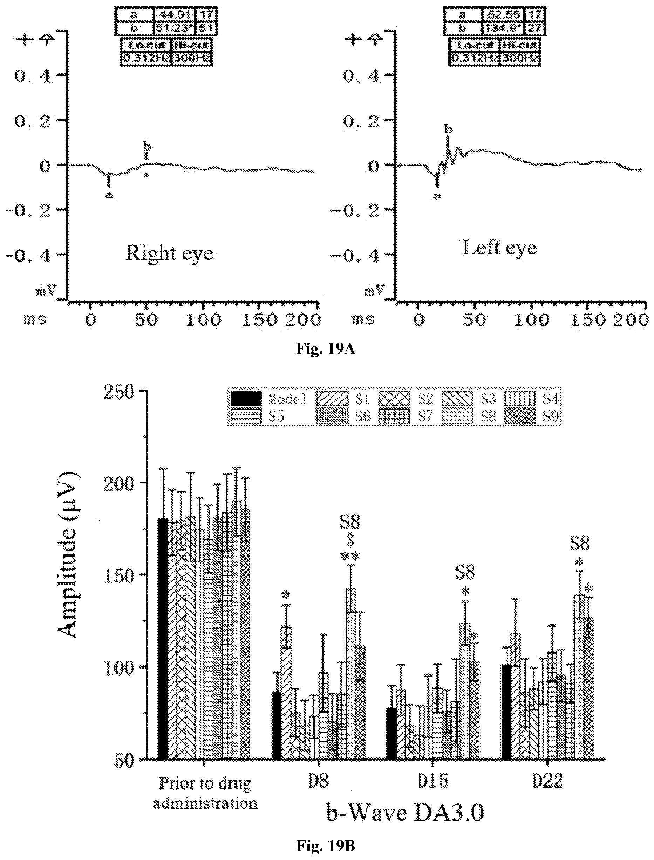

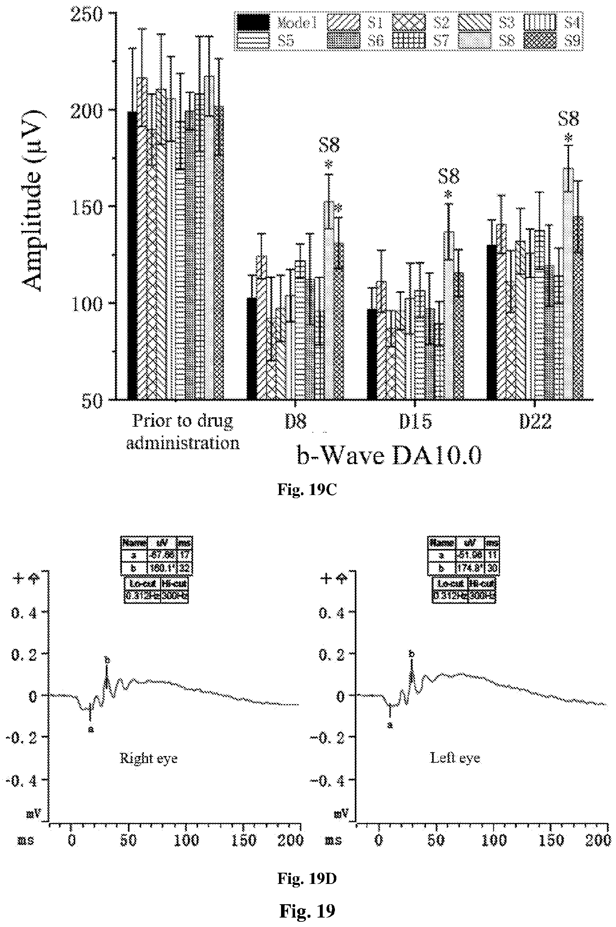

Fig. 19 shows the effects of different drugs on rat ERG DA3.0 and DA10.0 in ischemia reperfusion animal model; Fig. 19A shows exemplary ERG (DA3.0) in the left eye (normal eye) (right panel) and right eye (modeling eye) (left panel) of rats in model control group on 7th day (D8) after modeling; Figs. 19B and 19C show the results of b-wave amplitude of full field visual ERG DA3.0 and DA10.0 in the right eye of rats in each group respectively; Fig. 19D shows exemplary ERG DA3.0 in the left eye (normal eye) (right panel) and the right eye (modeling and drug administration eye) (left panel) of rats in S8 drug administration group on 7th day after administration (D8); where, *: Compared with model control group, $P < 0.05$; **: Compared with model control group, $P < 0.01$; $: Compared with S9 drug administration group.

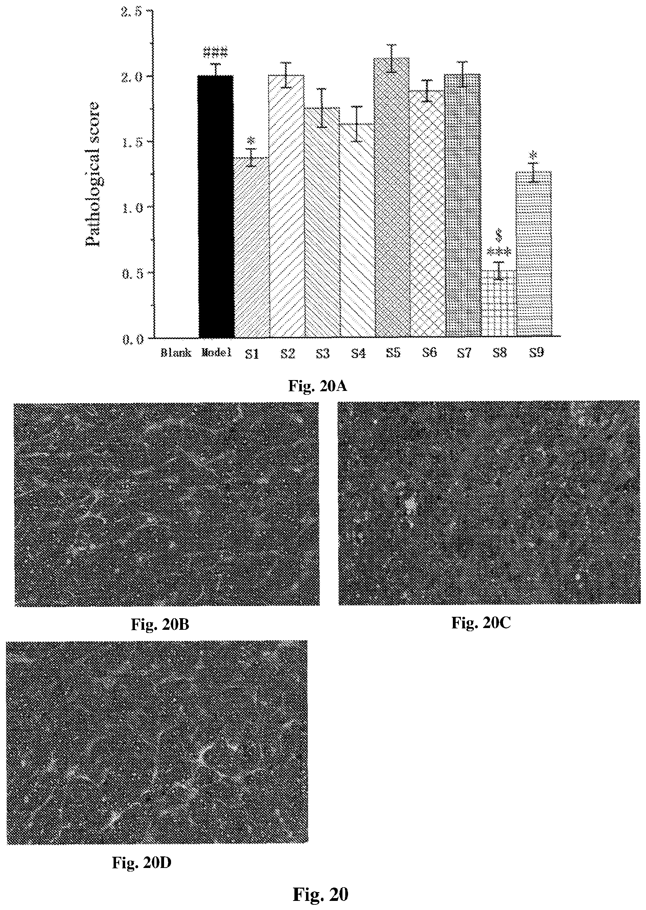

Fig. 20 shows the pathological changes of fundus induced by ischemia-reperfusion modeling and drug administration by staining ultrathin sections by toluidine blue; Fig. 20A, the result of pathological scores; Fig. 20B, exemplary images of the left eye as blank control; Fig. 20C, exemplary images of the right eye in model control group; Fig. 20D, exemplary images of the right eye in S8 drug administration group; where, ###: Compared with blank control group; *: Compared with model control group, $P < 0.05$; ***: Compared with model control group, $P < 0.001$; $: Compared with S9 drug administration group.

## Detailed Description of the Embodiments

The present invention may be understood more readily by reference to the following detailed description of certain embodiments of the invention.

As used herein, "administering" means oral ("po") administration, administration as a suppository, topical contact, intravenous ("iv"), intraperitoneal ("ip"), intramuscular ("im"), intralesional, intrahippocampal, intracerebroventricular, intranasal or subcutaneous ("sc") administration, or the implantation of a slow-release device e.g., a mini-osmotic pump or erodible implant, to a subject. Administration is by any route including parenteral and transmucosal (e.g., oral, nasal, vaginal, rectal, or transdermal). Parenteral administration includes, e.g., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, etc.

The terms "systemic administration" and "systemically administered" refer to a method of administering a compound or composition to a mammal so that the compound or composition is delivered to sites in the body, including the targeted site of pharmaceutical action, via the circulatory system. Systemic administration includes, but is not limited to, oral, intranasal, rectal and parenteral (i.e. other than through the alimentary tract, such as intramuscular, intravenous, intra-arterial, transdermal and subcutaneous) administration, with the proviso that, as used herein, systemic administration does not include direct administration to the brain region by means other than via the circulatory system, such as intrathecal injection and intracranial administration.

As used herein, the terms "treating" and "treatment" refer to delaying the onset of, retarding or reversing the progress of, or alleviating or preventing either the disease or condition to which the term applies, or one or more symptoms of such disease or condition.

The terms "patient," "subject" or "individual" interchangeably refers to a mammal, for example, a human or a non-human mammal, including primates (e.g., macaque, pan troglodyte, pongo), a domesticated mammal (e.g., felines, canines), an agricultural mammal (e.g., bovine, ovine, porcine, equine) and a laboratory mammal or rodent (e.g., rattus, murine, lagomorpha, hamster, guinea pig).

The present invention provides an L-N-isobutyryl cysteine (L-NIBC)-bound gold cluster molecule. The L-NIBC-bound gold cluster molecule comprises a gold core and a ligand L-NIBC bound to the gold core. The L-NIBC-bound gold cluster molecule has a formula of $Au_{18}(L\text{-}NIBC)_{14}$.

The present invention provides the L-NIBC-bound gold cluster molecule $Au_{18}(L\text{-}NIBC)_{14}$ for use of treatment of a disease in a subject. The disease includes multiple sclerosis, Alzheimer's disease (AD), liver cirrhosis, diabetes, Parkinson's disease (PD), and glaucoma.

The present invention provides a composition for use of treatment of a disease in a subject. The disease includes multiple sclerosis, Alzheimer's disease (AD), liver cirrhosis, diabetes, Parkinson's disease (PD), and glaucoma.

The present invention provides a compound for use in a a method for treatment of a disease in a subject by administering a composition to the subject. The disease includes multiple sclerosis, Alzheimer's disease (AD), liver cirrhosis, diabetes, Parkinson's disease (PD), and glaucoma.

In certain embodiments, the composition comprises the L-NIBC-bound gold cluster molecule $Au_{18}(L\text{-}NIBC)_{14}$.

In certain embodiments, when the L-NIBC-bound gold cluster molecule $Au_{18}(L\text{-}NIBC)_{14}$ is used as a component in a mixture of gold cluster molecules in the composition, the L-NIBC-bound gold cluster molecule $Au_{18}(L\text{-}NIBC)_{14}$ in the mixture has a chromatographic content of at least 7%, 8%, 9%, 10%, 11%, 12%, 13%, 14%, 15%, 16%, 17%, 18%, 19%, 20%, 21%, 22%, 23%, 24%, 25%, 26%, 27&, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

In certain embodiments, the composition further comprises a pharmaceutically acceptable excipient. In certain embodiments, the excipient is phosphate-buffered solution, or physiological saline.

The present invention provides a use of the L-NIBC-bound gold cluster molecule $Au_{18}(L\text{-}NIBC)_{14}$ for manufacturing a medication for the treatment of multiple sclerosis, Alzheimer's disease (AD), liver cirrhosis, diabetes, Parkinson's disease (PD) or glaucoma.

In certain embodiments, the dosage of the L-NIBC-bound gold cluster molecule $Au_{18}(L\text{-}NIBC)_{14}$ is at least 0.001 mg/kg/day, 0.005 mg/kg/day, 0.01 mg/kg/day, 0.05 mg/kg/day, 0.1 mg/kg/day, 0.5 mg/kg/day, 1 mg/kg/day, 2 mg/kg/day, 3 mg/kg/day, 4 mg/kg/day, 5 mg/kg/day, 6 mg/kg/day, 7 mg/kg/day, 8 mg/kg/day, 9 mg/kg/day, 10 mg/kg/day, 15 mg/kg/day, 20 mg/kg/day, 30 mg/kg/day, 40 mg/kg/day, 50 mg/kg/day, 60 mg/kg/day, 70 mg/kg/day, 80 mg/kg/day, 100 mg/kg/day, 200 mg/kg/day, 300 mg/kg/day, 400 mg/kg/day, 500 mg/kg/day, 600 mg/kg/day, 700 mg/kg/day, 800 mg/kg/day, 900 mg/kg/day, or 1000 mg/kg/day. The effective dosage for each disease can be ascertained by routine clinical trials.

The following examples are provided for the sole purpose of illustrating the principles of the present invention;

**Embodiments**

**Embodiment 1. Sample preparation**

**1.1. Protocol I**

Under the conditions of ice bath and stirring, 2500ml of anhydrous methanol solution of chloroauric acid was added to the reactor, in which the content of chloroauric acid was 5g, and then 500ml of acetic acid was added. After sufficient cooling, 500ml of methanol solution of L-N-isobutyryl cysteine (L-NIBC), in which the content of L-NIBC was 36g. After 60 minutes of reaction, 1500ml of ethanol solution of $NaBH_4$ was added with stirring, in which the content of $NaBH_4$ was 40g. At this time, the solution would quickly turn into brown black and a large amount of gas would emerge. After 30 minutes of reaction, 5000ml of acetone was added to terminate the reaction and precipitate the product. After centrifuging with a centrifuge, took the lower precipitate and discarded the upper supernatant. The precipitate obtained by centrifugation was dissolved in 500ml of ultrapure water and aged at room temperature 25°C and neutral conditions for 7 days. Then dialyzed in ultrapure water with a dialysis bag with a retained molecular weight of 500 Daltons, and changed the water every day. After 7 days, untied the dialysis bag and collected the solution in the bag. The collected solution was freeze-dried to obtain gold clusters crude products.

Gold clusters crude products may contain many gold cluster molecules with different molecular formulas; however, what gold cluster molecules are actually present in the gold clusters crude products is completely unknown; as for the actual content or its therapeutic effect of a specific gold cluster molecule in the gold clusters crude products, there is no way to know; these unknown factors cause great difficulties for the druggability and drug application of gold clusters crude products. Therefore, it is necessary to separate the different components contained in gold clusters crude products. For this purpose, the above gold clusters crude products were dissolved in water, and separated by preparative chromatography, and fractions with different retention times were collected according to outflow time. Each fraction was dialyzed with 500 Dalton dialysis bag respectively, and a series of gold clusters samples were obtained after freeze-drying; some samples were selected, and the molecular formulas of the main component in each sample were determined by ultra-high resolution magnetic resonance mass spectrometry (Bruker Daltonics, Solarix 7.0T ESI) combined with other methods. Their molecular formulas are as follows:

Sample 1 (S1): $Au_{25}(L\text{-}NIBC)_{18}$ as the main component;
Sample 2 (S2): $Au_4(L\text{-}NIBC)_4$ as the main component;
Sample 3 (S3): $Au_7(L\text{-}NIBC)_5$ as the main component;
Sample 4 (S4): $Au_{10}(L\text{-}NIBC)_6$ as the main component;
Sample 5 (S5): $Au_5(L\text{-}NIBC)_5$ as the main component;
Sample 6 (S6): $Au_{11}(L\text{-}NIBC)_9$ as the main component;

Sample 7 (S7): $Au_{13}(L\text{-}NIBC)_9$ as the main component;
Sample 8 (S8): $Au_{18}(L\text{-}NIBC)_{14}$ as the main component;

For the convenience of comparison, gold clusters crude product was included in the experiments, and designated as:
Sample 9 (S9): a mixture of three batches of gold clusters crude products.

## 1.2. Protocol II

Under the conditions of ice bath and stirring, 1300ml of anhydrous methanol solution of chloroauric acid was added to the reactor, in which the content of chloroauric acid was 5 grams, and then 200ml of acetic acid was added; added 300ml of methanol solution of L-N-isobutyryl cysteine (L-NIBC), in which the content of L-NIBC was 12 grams; added 1ml ultrapure water; after 30 minutes of reaction with stirring, added 500ml of methanol solution of $NaBH_4$, in which the content of $NaBH_4$ was 18 grams; at this time, the solution would quickly turn into brown black and a large amount of gas would emerge; after 30 minutes of reaction, added 3000m1 of acetone to terminate the reaction and precipitate the product. After centrifuging with a centrifuge, took the lower precipitate and discarded the upper supernatant. The precipitate obtained by centrifugation was dissolved in 500ml of ultrapure water, and aged for 7 days at 25°C under acidic conditions. After centrifugation, the precipitates were discarded. Then dialyzed the supernatants in weak acidic water with a dialysis bag with a retained molecular weight of 500 Daltons, and changed the water every day. After 7 days, untied the dialysis bag and collected the solution in the bag. The collected solution was freeze-dried to obtain gold clusters crude products. The fractionation and determination of the molecular formulas of the main component in samples were performed as described in the above 1.1 protocol I. The results showed that $Au_{18}(L\text{-}NIBC)_{14}$ was not detected.

## Embodiment 2. Characterization of S8 ($Au_{18}(L\text{-}NIBC)_{14}$)

The molecular formula $Au_{18}(L\text{-}NIBC)_{14}$ is a typical molecular formula of S8 given by ultra-high resolution mass spectrometry. Because of the existence of carboxyl groups in L-NIBC, in different solutions or solvents, different numbers of hydrogen ions may be replaced by corresponding metal cations or other cations due to different pH values and the existence of metal cations or other cations in solutions or solvents.

Fig. 1 shows the size exclusion chromatography (SEC) and mass spectrum of S8 ($Au_{18}(L\text{-}NIBC)_{14}$). As shown in Fig. 1A, the SEC spectrum of S8 ($Au_{18}(L\text{-}NIBC)_{14}$) reveals a purity up to 99.54%. Fig. 1B shows the large-scale ESI mass spectrum of S8 ($Au_{18}(L\text{-}NIBC)_{14}$)) shows that the spectrum is very clean, indicating that there are few impurities. Figs. 1C-1F show the mass spectrum isotope peaks of S8 ($Au_{18}(L\text{-}NIBC)_{14}$) with different mass charge ratio (M/z).

Fig. 2 shows a typical UV-Vis absorption spectrum of S8 ($Au_{18}(L\text{-}NIBC)_{14}$). As shown in Fig. 2, an apparent absorption peak is present in the range of 500-700nm.

Fig. 3 shows the infrared absorption spectra of S8 ($Au_{18}(L\text{-}NIBC)_{14}$) (Upper spectrum) and ligand L-NIBC (Lower spectrum). It can be seen that the thiol group (-SH) absorption characteristic peak of ligand L-NIBC located near 2500 $cm^{-1}$ is no longer visible in S8 (shown in the box), while the characteristic absorption peaks of other functional groups still exist, indicating that ligand L-NIBC in S8 binds to gold atoms through thiol group.

For investigating the hydrophilic/lipophilic properties of S8 drugs in aqueous solutions with different pH values, 10mg S8 drug was dissolved in 10ml of aqueous solutions with different pH values (ultrapure water was added 5M HCl to adjust its pH values in the range of 1-7, and the specific pH values were measured by a pH meter; the measured pH value is defined as the pH value of initial aqueous solution); then, 10ml n-octanol was added on top of the aqueous solutions respectively; the aqueous solution and n-octanol were mixed fully by shaking, and then stood still until the n-octanol phase and aqueous phase were separated into two layers. The weight ratio concentration (ppm) of gold in the upper n-octanol phase and the lower aqueous phase was measured by graphite furnace atomic absorption spectrometer, and the lipid-water partition coefficient P was calculated according to equation (1):

$$\log P = \log(Co/CW) \quad (1)$$

where Co and Cw respectively represent the concentration of S8 drug in oil phase and water phase.

As shown in Fig. 4, at high pH values of initial aqueous solutions (e.g. pH > 3.4), S8 drug is mainly dissolved in water phase, while the content in n-octanol phase is very low, and its lipid-water partition coefficient is less than -2 (P<0.01). However, below pH 3.4 of initial aqueous solution, with the decrease of pH value, the content in the aqueous phase decreases rapidly, while the content in the n-octanol phase increases sharply. When the pH value of initial aqueous solution decreases to 3.0, the lipid-water partition coefficient reaches about 1 (P= 9.7), and when the pH value of initial aqueous solution is 2.8, the lipid-water partition coefficient reaches 1.31 (P=20.5). It can be seen that S8 drug has achieved a significant reversal of hydrophilic/lipophilic properties in a very narrow range of pH values of initial aqueous solution (as shown in the middle area of the dotted line above). These properties are very different from conventional drugs, which may be of great significance in the formulation design of drug for clinical applications.

**Embodiment 3. Animal experiment of multiple sclerosis (Experimental autoimmune encephalomyelitis (EAE) model)**

**3.1. EAE model experiments**

C57BL/6N female mice, 7-9 weeks old. Animals were housed and maintained in compliance with the regulations.

On Day 0, mice were grouped randomly by body weight, and then injected subcutaneously with 200µl of myelin oligodendrocyte glycoprotein (MOG) peptide in complete Freund's adjuvant (CFA) (Hooke Laboratories) into the right and left flank, 100µl per site. Pertussis toxin (PTX) was injected by i.p at 0 and 48 hours after MOG immunization. The above AuCs samples were respectively given from day 1 to day 28 according to Table 1. The animals (n=10) were scored daily for clinical signs of EAE.

Hematoxylin eosin staining (HE) is one of the most commonly used staining methods for tissues and cells. Hematoxylin staining solution has high affinity for the nucleus, making the nucleus dyed blue, and eosin staining solution has high affinity for the cytoplasm, making the cytoplasm pink or red. 3 mice from each group were taken, and tissues were frozen at -80°C refrigerator; for tissue section, the frozen tissues were taken out, and put into the frozen slicer (box temperature, -20°C) and balanced for 15 min; the tissue section was 10µm.

HE staining as follows: after the behavioral test, the mice were anesthetized and perfused into the heart, then the mouse brain was taken out and fixed with 4% paraformaldehyde. After gradient dehydration, the frozen coronal sections were pasted with adhesive slides. The slides pasted with brain slices were put into oven at 37°C for 15 min; the slides were stained with hematoxylin staining solution for 8 min, washed off the excess dye in tap water for 10 min, and rinsing the slides with purified water for a few seconds; the slides were differentiated by differentiation solution for 30s (optional), and rinsed with tap water for 10 min; then the slides were stained with eosin for 1 min, rinsed with tap water for 10 min, 70% ethanol for 10s, 80% ethanol for 10s, 90% ethanol for 10s, 100% ethanol for 10s, xylene for 5 min, fresh xylene for 5 min, and sealed with neutral gum seal; microscopic observation showed that the nucleus was blue and the cytoplasm was pink.

Table 1. Grouping and dosages

| Group | Model | Drug administration | Dosage (mg/kg) | Dosage volume (mL/kg) | Route | Animal number |
|-------|-------|---------------------|----------------|------------------------|-------|---------------|
| 1 | Blank Control | Normal saline | - | 10 | i.p. | 10 |
| 2 | EAE | Normal saline | - | 10 | i.p. | 10 |
| 3 | EAE | S1 drug | 10 | 10 | i.p. | 10 |
| 4 | EAE | S2 drug | 10 | 10 | i.p. | 10 |
| 5 | EAE | S3 drug | 10 | 10 | i.p. | 10 |
| 6 | EAE | S4 drug | 10 | 10 | i.p. | 10 |
| 7 | EAE | S5 drug | 10 | 10 | i.p. | 10 |
| 8 | EAE | S6 drug | 10 | 10 | i.p. | 10 |
| 9 | EAE | S7 drug | 10 | 10 | i.p. | 10 |
| 10 | EAE | S8 drug | 10 | 10 | i.p. | 10 |
| 11 | EAE | S9 drug | 10 | 10 | i.p. | 10 |
| Note: EAE denotes experimental autoimmune encephalomyelitis; i.p. means intraperitoneal administration. | | | | | | |

Table 2. Behavioral clinical scoring rubric for EAE

| Score | Clinical findings |
|-------|-------------------|
| 1 | Loss of tail tonicity |
| 2 | Mild hind limb weakness |
| 3 | Partial hind limb paralysis |

(continued)

| Score | Clinical findings |
|---|---|
| 4 | Complete hind limb paralysis |
| 5 | Complete hind limb paralysis with forelimb weakness or moribund |

### 3.2. Results and analysis

Fig. 5 shows the test results of the behavioral clinical scores of EAE model mice in the drug administration groups represented by S1, S2, S8 and gold clusters crude product S9. As shown in Fig. 5, S1 ($Au_{25}$(L-NIBC)$_{18}$) and S2 ($Au_4$(L-NIBC)$_4$) drugs did not significantly improve the behavioral clinical scores of EAE model mice. However, all mice in S8 ($Au_{18}$(L-NIBC)$_{14}$) drug group were almost the same as normal mice in the first 13 days, and showed weak increase of the behavioral clinical scores from 14th day, and in the whole experimental period, the behavioral clinical scores were less than 1. In comparison with model group mice, there was huge improvement (from 9th day, $P$ <0.001), indicating that S8 drug had a magical effect on inhibiting the behavioral disorder of mice caused by MOG drug. In comparison with S8 drug group, S9 (gold clusters crude product) drug administration group only showed significant difference than model control group until 19th day ($P$ < 0.05), and the behavioral clinical score at the highest point reached above 2 points; meanwhile, although the score improved rapidly in the follow-up administration and decreased to less than 1 point from 22nd day, it still showed that the curative effect of S9 drug was significantly less than that of S8 drug. The test results of the other five drugs (S3-S7) were similar to those of S1 and S2, and no obvious pharmacodynamic effect was found, which will not be described in detail herein.

Fig. 6 shows the test results of HE staining. In EAE animal model, a large number of immune cells will invade bone marrow. The invasion of immune cells into bone marrow can be seen by HE staining. Fig. 6A shows the statistical results of histological score of exemplary drug administration groups. As shown in Fig. 6A, S8 drug can significantly reduce the increase of histological score caused by EAE modeling ($P$ < 0.01, * *), and S9 (gold clusters crude product) drug administration can also significantly reduce the score ($P$ < 0.05, *), but it is significantly less effective than S8 drug, and S1 and S2 have no obvious effect. Drugs S3-S7 are similar to drugs S1 and S2, no apparent effects; they will not be described in detail herein. Fig. 6B-6F are exemplary HE staining images of blank control group, model control group, S1 administration group, S8 administration group and S9 administration group respectively. It can be seen that a large number of immune cells invaded the bone marrow in the model control group (Fig. 6C), as shown in the box, while there was no such phenomenon in the mice of the blank control group (Fig. 6B). Fig. 6D is an exemplary HE staining image of S1 drug administration group; a large number of immune cell invasion can be seen, as shown in the box, which is similar to that of model control group mice. S8 drug Administration (Fig. 6E) can significantly reduce the invasion of immune cells into bone marrow, and the infiltration of immune cells is almost invisible, indicating that the effect is excellent. S9 drug administration group had a certain therapeutic effect, but a certain degree of immune cell invasion could still be seen in the bone marrow (Fig. 6F). Similar to S1, S2-S7 did not significantly inhibit the invasion of immune cells into bone marrow. These results are consistent with the behavioral results shown in Fig. 5.

The above results demonstrate that S8 drug ($Au_{18}$(L-NIBC)$_{14}$) can significantly improve intracerebral inflammation and behavioral clinical score of EAE model mice, and has obvious advantages compared with other drugs in this experiment. The effects of S8 drug are superior to S9 (gold clusters crude product) drug. Therefore, it has broad application prospects in multiple sclerosis and other immune related diseases.

Because gold cluster molecule $Au_{18}$(L-NIBC)$_{14}$ showed unique efficacy, we detected the content of $Au_{18}$(L-NIBC)$_{14}$ in S9 sample (gold clusters crude product). According to the chromatographic results (detection wavelength 220 nm), the purity of $Au_{18}$(L-NIBC)$_{14}$ chromatographic peak was calculated as 2.8% by area normalization method. Researches showed that the chromatographic content of $Au_{18}$(L-NIBC)$_{14}$ in gold clusters crude products varied due to the influence of preparation methods, preparation conditions and preparation batches. Under the preparation methods and conditions of the protocol I of the present study, the chromatographic content of $Au_{18}$(L-NIBC)$_{14}$ were in the range of 1% - 4% depending upon the different preparation batches.

The fact that the efficacy of S9 drug (gold clusters crude product) is significantly less than that of S8 drug indicates that the content of gold cluster molecule $Au_{18}$(L-NIBC)$_{14}$ is one of the important factors affecting the efficacy of the samples. To further investigate this point, we added different quantities of S8 drug into S9 drug (gold clusters crude product) to obtain a series of S9+S8 drugs so that the chromatographic content of gold cluster molecule $Au_{18}$(L-NIBC)$_{14}$ was different in each of the S9+S8 drugs, and then tested with the EAE model. The results showed that when the chromatographic content of gold cluster molecule $Au_{18}$(L-NIBC)$_{14}$ in an S9+S8 drug reached or exceeded 7%, such a drug showed great efficacy, indicating that L-NIBC-AuCs product with 7% or higher chromatographic content of gold cluster molecule $Au_{18}$(L-NIBC)$_{14}$ shall be effective in treating MS.

Fig. 7 shows the EAE model drug test behavioral clinical scores of S8 drug, S9 drug, and S9+S2 mixture drug, where the chromatographic content of gold cluster molecule $Au_{18}(L\text{-}NIBC)_{14}$ in the S9+S2 mixture drug was about 21%. It can be seen that after adding a small amount of S8 to the gold clusters crude product S9, the therapeutic effect on EAE model mice has increased significantly, which is almost no different from S8.

**Embodiment 4. Alzheimer's disease (AD) animal model experiments**

**4.1. Experiments of APP/PS1 double transgenic AD model mice**

**4.1.1. Methods**

Male C57BL/6 germline APP/PS1 transgenic AD model mice were randomly divided into model control group, S1 drug administration group, S2 drug administration group, S3 drug administration group, S4 drug administration group, S5 drug administration group, S6 drug administration group, S7 drug administration group, S8 drug administration group and gold clusters crude product S9 drug administration group. At the same time, C57BL/6 wild-type mice of the same age group were set as normal control group. Each group contained 15 mice. S1, S2, S3, S4, S5, S6, S7, S8 and S9 groups were injected intraperitoneally with normal saline solution of corresponding drugs once a day, with a dose of 10mg/kg mouse body weight and an injection volume of 100μl. Mice in model control group and normal control group were intraperitoneally injected with the same volume of normal saline.

When the mice were 3-months old, drug administration was commenced. After 180-days continuous administration, the cognitive and memory functions of all animals were assayed by Morris water maze test.

Positioning navigation experiment: Morris water maze test system consisted of a circular water pool and an automatic video recording and analysis system. Cameras above the water pool were connected to a computer. The water maze consisted of a circular water pool with a diameter of 120cm and a height of 60cm, and a platform with a diameter of 9cm. The liquid level was 0.5cm higher than the platform and the water temperature was at $22 \pm 0.5°C$. White pigment was used to dye water to milky white. The positioning navigation experiment was used to measure the learning and memory abilities of mice in water maze, which lasted for 5 days. The water maze was divided into four quadrants in the four directions of E, W, S and N. The platform was placed in a fixed quadrant. The position of platform was fixed throughout the experiment. During training, the mice were gently put into water from 1/2 radian in different quadrants near the outer wall, with heads facing the pool wall. The time that the mice spent in climbing onto the hidden platform was recorded, or the experiment was stopped when the record time reached 60s. The mice were allowed to stay on the platform for 30 s after climbing onto the platform. If the mice failed to find the platform within 60s, the experimenters would guide the mice to climb onto the platform and let them stay on the platform for 30s. The escape latency of mouse platform searching was recorded by camera tracking system. After the experiment, every mouse was moved away and dried gently by a dryer. Each mouse was trained 4 times a day for 5 consecutive days, with 20 min intervals between training sessions.

Spatial probe test: After finishing the training on the 5th day, the platform was removed on the 6th day, the mice were gently put into the water from the farthest point away from the platform facing the pool wall, the 60s movement orbits of the mice were recorded by camera, and the times of mice crossing the platform, time of stay in the target quadrant and the swimming distance in the target quadrant were analyzed by a software.

Experimental results are expressed by $\bar{x} \pm SEM$. All data were processed by SPSS software (SPSS 21) and analyzed by Post-Hoc Dunnett test, $P < 0.05$ indicates that the difference is statistically significant. Kruskal Wallis H test and Mann Whitney U test were used for statistical analysis of data not belonging to normal distribution.

After the behavioral test, the mice were anesthetized by intraperitoneal injection of 7% chloral hydrate, the cardiac perfusion connection was established, the normal saline was used for rapid flushing for 7 minutes, and then the tissue was perfused with 4% chloral hydrate for 7 minutes. After perfusion, brain tissues were carefully collected and placed in 4% perfusion solution and stored at room temperature for future uses. Immunohistochemical method was used to detect the expression of Aβ1-40 in hippocampus and cortex: perfused tissue was dehydrated, hyalinized, waxed and embedded, and sliced with paraffin slicer. Gradient dewaxing with xylene and absolute ethanol. After antigen repair by microwave, the sections were incubated with hydrogen oxide, and the sections were blocked by serum for 30min. Added primary antibody (murine anti-Aβ1-40, 1:100) at room temperature, incubated at 4°C overnight (15h). Discarded the primary antibody, washed the sections with PBS, added HRP labeled Goat anti-mouse secondary antibody, and incubated at room temperature for 30min. After the slices were washed with PBS, the developer DAB was added for color development, and the slices were counterstained by Harris hematoxylin, dehydrated and sealed. Fluorescence microscope was used to take photos, and Image J was used for quantitative analysis of slices.

**4.1.2. Results**

Fig. 8 shows the effects of drug administration of S1, S2, S8 and S9 for 180 consecutive days on the performance of male mice in the Morris water maze test. As shown in Fig. 8A, during the training of positioning navigation experiments, compared with the normal control group (blank), the platform-seeking latency of model control group (model) mice was significantly higher than that of normal mice from day 2 to day 5 of training ($P < 0.05$, #; $P < 0.01$, ##). Compared with the model control group, the platform-seeking latency of S1 and gold clusters crude product S9 drug administration groups was evidently decreased, and showed significant differences at $4^{th}$ and $5^{th}$ days ($P < 0.05$, *). S8 drug administration resulted in even more significant decrease of platform-seeking latency in modelled mice, and in comparison with model control group, the difference was significant from $2^{nd}$ day to $5^{th}$ day ($P < 0.05$, *). In comparison with S9 drug administration group, the platform-seeking latency of S8 drug administration group was significantly lower at $3^{rd}$ day ($P < 0.05$, $). All these results demonstrated that the effects of S8 drug are better than that of S1 and gold clusters crude product S9 drug. S2 drug administration group did not show apparent decrease of platform-seeking latency.

As shown in Figs. 8B-8D, the results of the space probe test showed that compared with the normal control group mice, the model control group mice showed significantly reduced swimming distance (Fig. 8C) ($P < 0.01$, ##) and time spent in the target quadrant (Fig. 8D) ($P < 0.05$, #), and less platform-crossing times with no significant difference ($P > 0.05$). Compared with the model control group, S2 drug failed to increase the platform-crossing times of the mice (Fig. 8B), while the platform-crossing times in S1, S2 and S9 drug administration groups increased, but none showed significant difference ($P > 0.05$). For target quadrant swimming distance and target quadrant time spent (Fig. 8C and Fig. 8D), S1, S8 and S9 drugs can significantly improve these two values ($P < 0.05$, *), where the increase amplitude of S8 drug administration group is larger than that of S1 and S9 drug administration groups. However, S2 drug failed to significantly increase these two values.

These data show that although S1, S8 and gold clusters crude product S9 drugs all can significantly improve the cognitive and memory ability of AD model mice, S8 drug showed better therapeutic effects than S1 and S9 drugs. S2 drug showed no apparent effects.

The results of S3-S7 drugs for administration of 180 consecutive days are similar to that of S2 drug. In comparison with model control group, there was no apparent increase of platform-seeking latency during the training of positioning navigation experiments, or platform-crossing times, swimming distance in target quadrant and time spent in target quadrant in the space probe test, indicating that S3-S7 drugs showed no apparent effect on improvement of the cognitive and memory ability of AD model mice.

The results of immunohistochemical test further confirmed that S8 gold cluster molecule ($Au_{18}(L\text{-}NIBC)_{14}$) could significantly reduce the formation of A$\beta$1-40 fibrotic plaque in the brain of APP/PS1 double transgenic AD model mice. Fig. 9 shows the comparison of A$\beta$1-40 plaque numbers in terms of average optic density values in the hippocampus and cortex between model control group and S1, S2, S3 or S9 drug administration groups. As shown in Fig. 9, S8 drug can significantly reduce the number of A$\beta$1-40 plaque in the hippocampus and cortex; in comparison with model control group, the reduction is significant ($P < 0.01$, **). Gold clusters crude product S9 also had relatively good effects of reduction, and the number of A$\beta$1-40 plaque in the hippocampus and cortex was significantly lower than that of model control group ($P < 0.05$, *), but the reduction amplitude is significantly lower than that of S8 drug administration group ($P < 0.05$, $). S1 drug administration group was similar to S9 drug administration group, reducing the number of A$\beta$ fibrotic plaques in the brain, but the reduction amplitude is significantly lower than that of S8 drug administration group. These results demonstrated that S8 drug has excellent effects on reduction of AD intracerebral fibrotic plaques, superior to S1 drug and gold clusters crude product S9. However, S2 drug showed no therapeutic effect. S3-S7 drugs are similar to S2 drug, showing no therapeutic effects; they will not be described in detail herein.

### 4.2. Experiments of five-transgenic 5xFAD AD model mice

Conventional mouse models (such as the afore-mentioned APP/PS 1 double transgenic mouse model) are useful for the initial exploration and research of AD etiology and therapeutic drugs. However, due to the complex pathogenesis of AD, more in-depth research needs more appropriate animal models. 5xFAD is a relatively new APP/PS1 transgenic AD mouse model, which carries five familial gene mutations and has a variety of AD-related nerve damage and memory dysfunction. Compared with APP/PS1 double transgenic mouse model and other AD animal models, the characteristics of neuropathological lesions are more obvious, so it has been more and more widely recognized.

### 4.2.1. Methods

Model mice were 5xFAD male mice with C57BL/6 background. Mice for experiments were divided into model control group, S1 drug administration group, S2 drug administration group, S3 drug administration group, S4 drug administration group, S5 drug administration group, S6 drug administration group, S7 drug administration group, S8 drug administration group and gold clusters crude product S9 drug administration group. At the same time, C57BL/6 wild-type mice of the same age group were set as normal control group. Each group contained 15 mice. Mice in drug administration groups were injected intraperitoneally with normal saline solution of corresponding drugs once a day, with a dose of 10mg/kg mouse body weight and an injection volume of 100$\mu$l. When the mice were 2-months old, drug administration was commenced. Mice in model control group and normal control group were intraperitoneally injected with the same volume of normal saline following the same schedule for drug administration groups.

After 3-months consecutive drug administration, the cognitive and memory functions of all animals were assayed by Morris water maze test.

Tests of spatial learning capability

Morris water maze system consists of a water maze for mouse swimming and an automatic video recording and analysis system. Cameras above the water maze were connected to a computer. The water maze consists of a circular water pool with a diameter of 110cm and a height of 60cm. During the training of mouse swimming, the water in the water pool is darkened by black ink, and the water temperature is kept at $22 \pm 0.5°C$. The water maze is divided into North, South, West and East quadrants. A platform with a diameter of 9cm is placed at a fixed quadrant. The platform is 0.5cm lower than the water level.

Mice were trained to learn about the hidden platform in water maze for 5 days. During training, the mice were gently put into water in different quadrants with heads facing the pool wall. The time that the mice spent in finding and climbing onto the hidden platform was recorded, and the mice were allowed to stay on the platform for 30 s after climbing onto the platform. If mice could not find the platform within 60s, the training was stopped, and the experimenters would guide the mice to climb onto the platform and let them stay on the platform for 30s. The platform-seeking latency of mice was recorded by camera tracking system. After the experiment, every mouse was moved away and dried gently by a dryer. Each mouse was trained 4 times a day for 5 consecutive days, with 20 min intervals between training sessions.

Test of spatial memory capability (spatial probe test)

The swimming training was completed after 5 days. The probe test was commenced on the 6th day. The mice were gently put into the water from the farthest point away from the platform facing the pool wall, the 60s movement orbits of the mice were recorded by video camera, and their platform crossing times, time spent in the target quadrant and the swimming distance in the target quadrant were analyzed by software.

Experimental results are expressed by $\bar{x}\pm$SEM. All data were processed by SPSS software (SPSS 21) and analyzed by Post-Hoc Dunnett test, $P < 0.05$ indicates that the difference is statistically significant. Kruskal Wallis H test and Mann Whitney U test were used for statistical analysis of data not belonging to normal distribution.

Pathological analysis was conducted after the behavioral test. The mice were sacrificed by $CO_2$, and then the cardiac perfusion connection was established. The normal saline was used for rapid flushing for 5-10 minutes, and then the tissue was then fixed in 4% PFA overnight. Next day, the tissues were dehydrated, hyalinized, waxed and embedded in paraffin to form paraffin embedding block for slicing with a paraffin slicer. After slicing, gradient dewaxing the slices with xylene and absolute ethanol. Then amyloid plaques, astrocytes and microglia were detected by immunohistochemical method. After antigen repair by microwave, the sections were incubated with hydrogen oxide, and the sections were blocked by serum for 30min. Added primary antibody (murine anti-A$\beta$42, 1:100), GFAP and Iba 1 at room temperature. Discarded the primary antibody, washed the sections with PBS, added HRP labeled Goat anti-mouse secondary antibody, and incubated at room temperature for 30min. After the slices were washed with PBS, the developer DAB was added for color development, and the slices were counterstained by Harris hematoxylin, dehydrated and sealed. Fluorescence microscope was used to take photos, and Image J was used for quantitative analysis of slices.

### 4.2.2. Results

The results of behavioral study showed that from the second day to the fifth day of training, the platform-seeking latency of model control group mice was significantly higher than that of normal control group mice. Compared with the model control group, the platform-seeking latency of S8 drug administration group mice was significantly reduced, and there was a significant difference compared with the model control group from the third day. S1 and gold clusters crude product S9 drug administration groups showed reduced platform-seeking latency compared with model control group, but without statistical significance. No apparent reduction was observed in S2-S7 drug administration groups.

The spatial probe test also yielded similar results. S8 drug could significantly improve the platform crossing times, the swimming distance in the target quadrant and the time spent in the target quadrant. S1 and gold clusters crude product S9 drug administration groups showed somewhat reduction, but without statistical significance. S2-S7 drug administration groups showed no effect.

Fig. 10 shows the A$\beta$42 immunochemical staining results of fibrotic plaques in the intracerebral cortex and hippocampus of mice in the model control group and drug administration groups. As shown in Figs. 10A, 10B, 10C, and 10E, a large number of A$\beta$ plaques appeared in the above areas of the model control group mice at the age of 5 months. Compared with the model control group, the number of A$\beta$ plaques in the intracerebral cortex and hippocampus of S9 drug administration group mice was reduced to a certain extent, but without significant difference ($P > 0.05$). However, S8 drug reduced the number of Aβ plaques in the intracerebral cortex and hippocampus more than half ($P < 0.01$), indicating that S8 drug has excellent therapeutic effect, and the effect is much better that that of gold clusters crude product S9. Also, there is significant difference between gold clusters crude product S9 and S8 drug administration groups ($P < 0.05$, \$), further demonstrating the effect difference. Fig. 10D and 10F respectively show the exemplary images of immunohistochemical staining of cortex and hippocampus. S1 drug showed similar effect to S9 drug; there was somewhat reduction but without significant difference in comparison with model control group. S2 drug administration group showed no reduction in comparison with model control group, indicating of no effect. S3-S7 drug administration groups were similar to the S2 drug administration group without apparent effect.

Intracerebral inflammation is another important hypothesis for the pathogenesis of AD. Microglia and astrocytes are the main cells causing neuroinflammation. Iba1 and GFAP are respective markers for microglia and astrocytes. The expression increase of these proteins indicates the increase in the number of microglia and astrocytes and cell activation. Many studies have shown that the expression of Iba1 and GFA in the brain of AD mice is significantly increased. The activation of these cells can lead to massive production of cytokines such as interleukin (IL-1 β, IL-6), tumor necrosis factor (TNF) and reactive oxygen species, resulting in neurological dysfunction and death. Therefore, evaluating the effect of drugs on the counts of Iba1 positive (Iba1$^+$) and GFAP positive (GFAP$^+$) cells can show the effect of drugs on intracerebral inflammation related processes.

Fig. 11 shows the effects of drugs on the numbers of Iba1+ positive cells and GFAP$^+$ cells in the intracerebral cortex and hippocampus regions of 5xFAD transgenic AD model mice. Figs. 11A and 11B show the test results of immunohistochemical staining of Iba1$^+$ cells in the cortex and hippocampus of mice in the model control group and each drug administration group, respectively. Figs. 11C and 11D are the test results of immunohistochemical staining of corresponding GFAP$^+$ cells. It can be seen that S1 drug had no significant effect on the counts of Iba1$^+$ and GFAP$^+$ cells in intracerebral cortex and hippocampus, indicating that S1 drug has no significant inhibitory effect on brain inflammation. S2-S7 are similar to S1, no apparent inhibitory effect. Gold clusters crude product S9 reduced the numbers of Iba1+ positive cells and GFAP$^+$ cells in the intracerebral cortex and hippocampus regions, but no significant difference in comparison with model control group ($P > 0.05$). However, S8 drug showed a significant inhibitory effect on brain inflammation. As shown in Fig. 11A, compared with the model control group, S8 drug administration reduced the count of Iba1$^+$ cells in cortex by about 40%, showing significant difference ($P < 0.01$, \*\*). As shown in Fig. 11B, compared with the model control group, S8 drug administration significantly reduced the count of Iba1$^+$ cells in hippocampus by 27% ($P < 0.05$, \*). As shown in Fig. 11C and Fig. 11D, compared with the model control group, the count of GFAP$^+$ cells in cortex and hippocampus significantly decreased by 24% ($P < 0.05$, \*) and 15% ($P < 0.05$, \*) respectively. These results show that S8 drug has excellent inhibitory effect on AD-related intracerebral inflammation.

The above results show that S8 drug manifests excellent therapeutic effects in the five transgenic 5xFAD model mouse test, and can play a positive role in the treatment of AD at least from the three aspects of improving cognitive and memory behavior, inhibiting protein fibrosis and intracerebral inflammation. Combined with the test results of APP/PS1 double transgenic mouse model, it can be seen that S1 drug has certain therapeutic effect, and S9 drug is superior to S1 drug in term of the overall effect, but no comparison with S8 drug. S2-S7 drugs failed to show apparent therapeutic effects.

The above results show that the molecular structure of different gold cluster molecules containing the same ligand has a great impact on the application effect of gold cluster molecules in the prevention and treatment of Alzheimer's disease. For different gold cluster molecules containing L-NIBC, gold cluster molecule $Au_{18}(L\text{-}NIBC)_{14}$ (S8 drug) has extremely significant advantages over other seven gold cluster molecules and gold clusters crude product S9, and is more suitable for the development of drugs for the prevention and treatment of Alzheimer's disease.

Further research shows that gold clusters crude product S9 drug was added with S8 drug to obtain mixture drugs, and the therapeutic effect of the mixture drugs was significantly increased when the content of gold cluster molecule $Au_{18}(L\text{-}NIBC)_{14}$ in the mixture drugs reached 7%.

## Embodiment 5. Liver cirrhosis mouse model experiments

### 5.1. Methods

Modeling of mouse liver cirrhosis model

SPF male C57BL/6N mice, 6-8 weeks old and 16-20g body weight, were randomly divided based on their body weight into 11 groups ($n = 10$): blank control group, model group, S1 drug administration group, S2 drug administration group, S3 drug administration group, S4 drug administration group, S5 drug administration group, S6 drug administration group, S7 drug administration group, S8 drug administration group, and S9 drug administration group. Except for the blank control group, liver cirrhosis model of mice in other groups was prepared by the method of carbon tetrachloride ($CCl_4$)-induction treatment. The modeling protocol was as follows: (1) Each mouse was intraperitoneally injected with

10% CCl$_4$ (diluted with olive oil) at 7μl/g body weight, twice a week for a total of 8 weeks; mice of the blank control group were injected intraperitoneally with the same amount of olive oil solvent. (2) from the 6th week, two mice were selected and killed 48 hours after the last injection every week. The appearance of the liver was observed. After the appearance was in line with the characteristics of cirrhosis (the 8th week), the liver tissue was fixed with formalin. HE staining and Masson staining were used to evaluate the model of cirrhosis.

Drug administration

After the successful modeling, the mice of S1-S9 drug administration groups were given by intraperitoneal injection at 10 mg/kg respectively of the corresponding drugs; and the mice of the blank control group and the model group were given intraperitoneally physiological saline at 10 ml/kg. The administration was once a day for 20 consecutive days.

Biochemical assays

After the drug administrations were completed, blood was collected from mouse orbit, and sera were obtained for biochemical testing of albumin (albumin, ALB), total bilirubin (TBil), alanine aminotransferase (ALT), aspartate aminotransferase (AST) and monoamine oxidase (MAO) using Zhongsheng Beikong Kit and biochemical analyzer (Siemens). The detection method was performed in strict accordance with the kit instructions.

Pathological examination

HE staining

After euthanasia, the mouse liver tissue samples were fixed with 4% paraformaldehyde fixative for more than 48 h. After fixation, the liver samples were dehydrated with alcohol gradient and treated with xylene and ethanol. Then, the liver tissues were then dipped in wax and embedded. After the embedded material being trimmed, attached, and repaired, the liver tissues were sliced with a paraffin microtome, and the slices were with a thickness of 4μm. The main process of HE staining is as follows: After baked in the oven at 65°C, the slices were treated with xylene and dehydrated with gradient ethanol. The slices were sequentially stained with hematoxylin, blue color-enhancing solution, and 0.5% eosin, then treated with gradient ethanol and xylene and sealed with neutral gum. The fibrosis of liver tissue was observed with a microscope.

Masson staining

After baked, mouse liver tissue slices were dewaxed and dehydrated. After chromizing, the nucleus was stained with Regaud's hematoxylin staining solution. After washing with water, the slices were stained with Masson's Ponceau Red Acidic Fuchsin, and the slices were dipped in a 2% glacial acetic acid aqueous solution and differentiated with a 1% phosphomolybdic acid solution. After directly stained with aniline blue or light green solution, the slices were dipped in a 0.2% glacial acetic acid aqueous solution for a while, then transparentized with 95% alcohol, anhydrous alcohol and xylene, and then sealed with neutral gum. Liver tissue was observed with a microscope.

## 5.2. Results

The livers of mice in the model group were divided into round or oval masses of different sizes by proliferating fibrous septa. As shown in Fig. 12, the serum ALT, TBil, AST and MAO indexes increased significantly compared to that of the blank control group ($P < 0.01$, ##), and the serum ALB significantly decreased compared to the blank control group ($P < 0.05$, #), indicating that this experimental modeling was successful.

Fig. 12 shows the effects of drugs on serum levels of ALT (Fig. 12A), AST (Fig. 12B), TBil (Fig. 12C), MAO (Fig. 12D), and ALB (Fig. 12E) in liver cirrhosis model mice. The results show that gold clusters crude product S9 drug can significantly improve to the serum levels of ALT, AST, and TBil ($P < 0.05$, *) in the modeled mice, and S8 drug can significantly improve all five indexes in the sera of modeled mice ($P < 0.05$, *; $P < 0.01$, **). Compared with gold clusters crude product S9 drug administration group, S8 drug administration group showed more significant improvement of the levels of all five indexes, and there were significant differences in levels of ALT, AST and TBIL ($P < 0.05$, $), indicating that effects of S8 drug on improving the serum indexes in the liver cirrhosis model mice is significantly better than that of gold clusters crude product S9 drug. S1 drug had similar effects to S9 drug with improvement effect, but the amplitude of improvement is much lower that of S8 drug. S2 drug showed no apparent improvement of the five indexes in the blood of model mice. S3-S7 drugs are similar to S2 drug with no apparent improvement effect; they will not be described in detail herein. The above results showed that S8 drug has excellent therapeutic effects on liver cirrhosis in comparison with other drugs.

Fig. 13 shows the results of Masson staining experiments. Figs. 13A, 13B, 13C, 13D, and 13E respectively show the exemplary images of blank control group, model control group, S1 drug administration group, S8 drug administration group, and S9 drug administration group. As shown in Fig. 13A, the normal liver tissue from the mice of the blank control group had clear structure, intact liver lobules, neatly arranged hepatocyte cords with radial arrangement being centered on the central vein, normal nucleus of hepatocytes, and only a small amount of fibrous tissue in the catchment area. As shown in Fig. 13B, in the liver tissue of the model control group, the hepatocytes were disordered, balloon-like structures appeared, the liver lobules nearly disappeared, pseudolobules were abundantly formed, and a large number of proliferated protofibrils were present in the liver tissues, forming round- or oval-shaped fibrous septa.

As shown in Fig. 13D, compared with model control group, the liver tissues of S2 drug administration group mice manifested extremely significant recovery from liver damages, showing clear structure, intact liver lobules, neatly arranged hepatocyte cords, no observable fibril proliferation and pseudolobules, and no apparent difference than normal liver tissue, indicating extremely significant pharmacodynamics.

As shown Fig. 13E, gold clusters crude product S9 drug administration group also showed relatively significant effect, but compared with blank control group, certain amounts of fibril proliferation and pseudolobule structures are present. Combined with the above results of serum indexes, it shows that the comprehensive therapeutic effect of S8 drug is evidently better than that of S9 drug. As shown in Fig. 13C, S1 drug is similar to S9 drug; while S1 drug had certain therapeutical effects, it is still inferior to S8 drug. S2-S7 drugs failed to show therapeutic effect, where the hepatocytes were disordered, the liver lobules nearly disappeared, pseudolobules were abundantly formed, and a large number of proliferated protofibrils were present in the liver tissues, forming round- or oval-shaped fibrous septa; these are no significant different in comparison with model control group.

Fig. 13F shows the results of the effects of S1, S2, S8 and gold clusters crude product S9 drugs on the tissue histopathological scores of liver cirrhosis. It can be seen that the tissue histopathological scores of model control group increases from 0.5 to $3.35 \pm 0.42$ and shows extremely significant difference in comparison with blank control group ($P$ <0.001, ###). The scores of S8 drug administration groups were significantly decreased to $1.33 \pm 0.25$ in comparison with model control group ($P < 0.01$, **). Gold clusters crude product S9 drug administration group showed significant decrease in comparison with model control group ($P$ <0.05, *), but the decrease amplitude is 49% lower than that of S8 drug administration group, and there is significant difference between them ($P$ <0.05, \$). These results demonstrated that S8 drug has excellent protection effects on liver damages caused by liver cirrhosis, and therapeutic effects are significantly better than gold clusters crude product S9 drug. S1 drug is similar to S9 drug, having therapeutic effect but much less than S8 drug. S2 drug showed no apparent effect. The results of S3-S7 drug administration groups were similar to that of S2 drug administration group and had no obvious efficacy, which will not be described in detail herein. HE staining results are consistent with Masson staining results, and will not be described in detail herein.

It can be seen from the above results that the pathological test results are consistent with the biochemical test results, which collectively shows that gold cluster molecule $Au_{18}(L\text{-}NIBC)_{14}$ (S8 drug) has significant therapeutic effect on liver cirrhosis, and is significantly better than that of gold clusters crude product S9 drug and other gold clusters drugs. Gold cluster molecule $Au_{18}(L\text{-}NIBC)_{14}$ (S8 drug) is more suitable for the development of drugs for the treatment (prevention and cure) of liver cirrhosis.

Further research shows that gold clusters crude product S9 drug was added with S8 drug to obtain mixture drugs, and the therapeutic effects of the mixture drugs were significantly increased when the content of $Au_{18}(L\text{-}NIBC)_{14}$ in the mixture drugs reached 7%.

### Embodiment 6. Diabetes animal model experiments

### 6.1. Methods

### 6.1.1. Diabetes modeling, drug administration, and blood glucose test

The present experiments employed two protocols; one was that drug administration was conducted during modeling, and the other was that drug administration was performed after the modeling was successfully completed. The protocols were respectively as follows:

Protocol I. Simultaneous modeling and drug administration

SPF-grade C57BL/6 male mice were randomly divided into blank control group, model control group, S1 drug administration group, S2 drug administration group, S3 drug administration group, S4 drug administration group, S5 drug administration group, S6 drug administration group, S7 drug administration group, S8 drug administration group and S9 drug administration group according to fasting blood glucose level (n=12/group). The mouse model of diabetes was established by feeding with high-fat diet and small dose of streptozotocin, and the drug administration was commenced on the first day of modeling. First, mice in each group were given laboratory standard maintenance feed for 1 week; after 1 week, except for the blank control group, other groups were fed with high fat diet for 3 weeks. After 3 weeks, the mice in model control group and S1~S9 drug administration groups were intraperitoneally injected with streptozotocin (100mg/kg) for 5 consecutive days, resulting in further injury to the pancreas and a model of type 2 diabetes. The blank control group was injected with the same amount of normal saline as the control. From the first day of feeding maintenance feed, S1-S9 drug administration groups were administered intraperitoneally at the dose of 10mg/kg body weight once a day, and the blank control group and model control group were injected intraperitoneally with the same amount of normal saline. This administration method lasted for 35 days until the end of the experiment. After modeling and drug administration test, all mice were fasting for 5 hours to detect point-0 blood glucose (fasting blood glucose), and then after intragastric administration of glucose (1.5mg/g body weight), the blood glucose levels were detected at 0.5h, 1h, 1.5h and 2h respectively.

Protocol II. Drugs being administered after successful modeling

Diabetes mouse model induced by high fat diet was used to study the effect of drugs on reducing blood glucose levels. SPF-grade B6 mice were randomly divided into blank control group (normal feeding), model control group, S1 drug administration group, S2 drug administration group, S3 drug administration group, S4 drug administration group, S5 drug administration group, S6 drug administration group, S7 drug administration group, S8 drug administration group and S9 drug administration group. The model control group and 9 drug administration groups were fed with high-fat diet at the age of 2 months. The mice in the blank control group were fed with standard laboratory feed. The feeding was for 3 months. At the age of 5 months, the blood glucose of mice was measured to determine whether the mice fed with high-fat diet had elevated blood glucose and disorder of glucose metabolism, so as to ascertain the success of modeling. There were two kinds of drug administration. One was acute administration; mice in the nine drug administration groups were injected intraperitoneally once (5mg/ kg), and the corresponding fasting blood glucose and glucose tolerance were measured 30 minutes later; the other was long-term administration; mice in the nine drug administration groups were injected intraperitoneally (5mg/kg/day) for 2 months. The blank control group and model control group were injected with the same amount of normal saline intraperitoneally. One or two months after the drug administration, all mice were starved overnight for 16 hours to detect fasting blood glucose), and then after intragastric administration of glucose (1.5mg/g body weight), the blood glucose levels were detected at 15min, 30min, 45min, 60min and 120min respectively.

### 6.1.2. HE staining

After completion of the above tests, animals in each group were euthanized, the animal pancreas was quickly collected, and fixed in 4% paraformaldehyde; then the fixed animal pancreatic tissue was put into embedding box, washed with running water for 30 minutes to remove fixing solution. The tissue was dehydrated by alcohol gradient (50%, 70%, 80%, 95%, absolute ethanol), and treated with environmental transparent agent. The transparent tissue was successively immersed in pure paraffin (I, II, III) for 1h each time, so as to embed the tissue and completely remove the remaining transparent agent. The embedded pancreatic tissue was sliced into $3\mu m$ sections with a paraffin slicer continuously; the slices were baked at 60°C for 1h for future use. Put the slices into xylene for 15min for dewaxing, repeating 3 times, then immersed the slices in gradient ethanol (100%, 85% and 75%) for 5 min respectively, and rinse with running tap water for 5 min to dewax the slices. The slices were stained in hematoxylin dye solution for 5 min, then the excess dye solution was washed off with tap water; 0.7% hydrochloric acid ethanol was used for color separation for 10s, and the slices were washed with tap water until the nucleus and nuclear chromatin were clear. Slices were dehydrated with 70% and 90% ethanol for 10 min, dyed with 0.5% eosin solution for 5 min, and the excess dye was washed off with tap water. The stained slices were dehydrated with 70%, 80%, 90% and 100% ethanol for 10s, hyalinized with transparent agent, and dried in ventilated place. After dropping an appropriate amount of neutral gum, sealed the slices with a cover glass. The pathological sections were observed and photographed by optical microscope.

### 6.1.3. Insulin immunohistochemistry

After completion of the tests of protocol I, $3\mu m$ pancreatic slices were successively obtained. After $3\mu m$ pancreatic slices were dewaxed, they were incubated with 3% $H_2O_2$ deionized water at room temperature for 5-10 min to block the endogenous peroxide, and washed with phosphate buffer (PBS) for 5 min×3 times. The slices were placed in antigen repair solution, and the antigens were repaired in microwave oven. After natural cooling, the slices were washed with PBS for 5min×3 times. 5% BSA blocking solution was added dropwise and the slices were incubated at 37°C for 30 min. Discarded the blocking solution, added insulin immunohistochemical primary antibody incubation solution (antibody ratio: 1:200), incubated at 37°C for 1-2 h, and rinsed with PBS for 5 min×3 times to remove the excess primary antibody.

Horseradish peroxidase-labeled secondary antibody incubation solution was added dropwise and the slices were incubated at 37°C for 30 min. Washed slices with PBS 5 min×3 times. The slices were colored with DAB, observed under the microscope to control the color development time, and washed with tap water to terminate the staining. The nuclei were counterstained with hematoxylin, rinsed with tap water, gradient dehydration with different concentrations of alcohol (75%, 85% and 100%), transparent agent and neutral gum sealing. The pancreatic tissue was observed by microscope, the expression of insulin was detected by immunohistochemistry and photographed. Three visual fields containing islets (400 times) were randomly selected from each slice. The average optical density of each visual field was measured quantitatively by using the software Image Pro Plus version 6.0, and the average value was taken as the average optical density of the slice.

## 6.2. Results

Fig. 14A shows the test results of fasting blood glucose levels from Protocol I with simultaneous modeling and drug administration. As shown in Fig. 14A, the fasting blood glucose of the model control group was $20.3 \pm 4.1$ mM, which was significantly higher than that of the normal mice in the blank control group ($11.5 \pm 2.1$ mM; $P < 0.01$, ##). The fasting blood glucose of S8 drug administration group was $13.1 \pm 2.3$ mM, which were significantly lower than that of the model control group ($P < 0.05$, *). The fasting blood glucose of S9 drug administration group was decreased to $15.1 \pm 2.9$ mM, which is significantly different from that of model control group ($P < 0.05$, *). The fasting blood glucose of S1 drug administration group was decreased to $16.7 \pm 1.7$ mM, which is not significantly different from that of model control group ($P > 0.05$). S2-S7 administration groups were at the same level as the model control group, and had no obvious effect.

Fig. 14B shows the test results of glucose tolerance levels from Protocol I with simultaneous modeling and drug administration, where 0h corresponds to the fasting blood glucose in Fig. 14A. The blood glucose values at each time point in the model control group were significantly higher than those in the blank control group ($P < 0.01$, ##), indicating that the modeling was successful. The fasting blood glucose and blood glucose values at different time points after glucose feeding in the S1 drug administration group were lower than those in the model control group, where the data of fasting blood glucose (0h), 0.5h and 2h ($P < 0.05$, *) are significantly different, indicating that S1 drug has certain hypoglycemic effect. S9 drug administration group is similar to S1 drug administration group, but the data of fasting blood glucose (0h), 0.5h, 1.5h and 2h are significantly different compared with the model control group ($P < 0.05$, *). The blood glucose values of S8 drug administration group at 0h, 0.5h, 1h, 1.5h and 2h were significantly lower than those of the model control group (0h, $P < 0.05$, *; remaining time points, $P <0.01$, **), and the decrease amplitude of blood glucose was much greater than that of S1 drug and gold clusters crude product S9 drug (in the blood glucose results of 0.5h-2.0h, the decrease amplitude of blood glucose reduction was nearly 2 times more). S2 drug administration did not significantly reduce blood glucose at all time points. The results of S3-S7 drugs were similar to those of S2 drug, and there was no obvious effect, which will not be described in detail herein.

Fig. 15 shows the results of fasting blood glucose and glucose tolerance from Protocol II with drug being differently administered after modeling. Fig. 15A shows the results of glucose tolerance after acute drug administration (for the convenience of comparison, only data of S2, S8 and S9 are shown), where 0 minute corresponds to fasting blood glucose. As shown in Fig. 15A, both fasting blood glucose and blood glucose data at other time points in the model control group are significantly higher than those in the blank control group ($P < 0.05$, #), indicating successful modeling. Among them, the 0h fasting blood glucose of the model control group was $10.8 \pm 2.3$mM, while that of the blank control group was $6.7 \pm 1.1$mM. Because of acute drug administration in each drug administration group, their fasting blood glucose was not significantly different than that of the model control group. For the gold clusters crude product S9 drug administration group, the blood glucose values at each time point after glucose feeding were lower than those in the model control group, and the results at 15 min were significantly different ($P < 0.05$, *). S1 drug administration group was similar to S9 drug administration group, which will not be described in detail herein. For S8 drug, the decrease amplitude of blood glucose at each time point relative to the model control group was greater than that in S9 drug administration group (the decrease amplitude was 39% to 2.2 times higher at 15 min-60 min), and there were significant differences at 15 min, 30 min, 45 min and 60 min in comparison with model control group ($P < 0.05$, *). S2 drug administration group showed the same levels at all time points as model control group. S3-S7 drug administration groups were similar to S2 drug administration group, at the same level as the model control group at each time point.

Fig. 15B and Fig. 15C are the results of fasting blood glucose from Protocol II after one-month or two-month consecutive drug administration respectively (for the convenience of comparison, only data of S2, S8 and S9 are shown). The fasting blood glucose of model control group was $12.2 \pm 0.7$ mM and $10.4 \pm 0.6$ mM respectively, which increased significantly compared with the data of the blank control group ($5.8 \pm 0.5$ mM and $5.5 \pm 0.3$ mM) ($P < 0.01$, ##). The values of S9 drug administration group decreased, but there was no significant difference compared with the model control group ($P > 0.05$). The decrease amplitude of S8 drug administration group was greater, and the values were decreased significantly to $9.8 \pm 0.3$ mM and $8.3 \pm 0.4$ mM respectively (decrease amplitude is respectively 35% and 3.2 times higher than that of S9 drug), and it was significant compared with the model control group ($P < 0.05$, *). S2 drug administration group was at a level similar with that of model control group, no apparent decrease. S3-S7 drugs were similar to S1 drug, and there was no significant decrease, which will not be described in detail herein.

Fig. 15D shows the results of glucose tolerance after two-month consecutive drug administration of S8 drug. As shown in Fig. 15D, the blood glucose values of S8 drug administration group at 0 min, 15 min, 30 min, 60 min and 120 min were significantly lower than those of model control group ($P < 0.05$, *), which verified the excellent hypoglycemic effect of S8 drug.

The above results mutually corroborate and collectively indicate that S8 drug shows excellent hypoglycemic effect, and the effect is significantly better than S1 drug and gold clusters crude product S9 drug. S2-S7 drugs had no obvious hypoglycemic effect.

Fig. 16 shows the results of HE staining and insulin immunohistochemistry from protocol I with simultaneous modeling and drug administration. Figs. 16A, 16B and 16C are exemplary immunohistochemical images of blank control group, model control group and S8 drug administration group respectively. The immunohistochemical results showed that the mice in each group could see the insulin positive signal, which was yellow to brown (Note: Brown was darker in the gray image, and yellow was lighter in the gray image). The main expression locations were islet cells. The insulin staining in the blank control group showed a strong brown positive signal (the color in the gray image was very dark, as indicated by the area confined by dotted lines in Fig. 16A); the insulin staining signal in the model control group was significantly weakened, and the color was significantly lighter to sparse light brown to yellow (the color in the gray diagram was lighter, as indicated by the area confined by dotted lines in Fig. 16B), while the mice in the S8 drug administration group were similar to the normal mice in the blank control group, and also showed strong brown positive signal (the color in the gray diagram was very dark, as indicated by the area confined by dotted lines in Fig. 16C). Fig. 16D shows the statistical results of the average optical density values of blank control group, model control group, and different drug administration groups. As shown in Fig. 16D, the expression of insulin in model control group was significantly lower than that in blank control group ($P < 0.05$, #), and the expressions of insulin in S1 and S9 drug administration groups were higher than that in the model control group (more apparent in S9 drug administration group), but the differences were not significant ($P > 0.05$), indicating that S1 and S9 have some protection effects on islets. Compared with model control group, S8 drug administration group had significantly higher insulin expression ($P < 0.05$, *), indicating that S8 drug has very good effects on protecting islets from damage or repairing islet damage. S2 drug administration group was at the same level of insulin expression as model control group, indicating that S2 drug did not have the function of protecting or repairing islets. S3-S7 drugs were similar to S2 drug, which will not be described in detail herein. The test results of drug administration after modeling in Protocol II were similar to those here. S8 drug also showed significant effect on protecting islets from injury or repairing islet injury (compared with model control group, $P < 0.01$). Although S9 also has certain effects that are significantly different from that of model control group ($P < 0.05$), it is much less effective than S8 drug.

On the other hand, HE staining results show that compared with the blank control group (Fig. 16E), the pancreas of the model control group (Fig. 16F) showed obvious histological changes, mainly manifested in the reduction of the number of islets, the severe reduction of islet volume, the significant reduction of the number of islet cells, and mild to moderate islet inflammation. In the S8 drug administration group (Fig. 16G), the tissue structure of islets was significantly improved compared with the model control group, and compared with the blank control group, there was no abnormal change, and no inflammatory cell infiltration in islets, which also shows the excellent protective effect of S8 drug on islets. Although the islet tissue structure of S9 drug administration group was also significantly improved compared with the model control group, the effects are much less apparent than that of S8 drug administration group; more specifically, the islet volume decreased to a certain extent, the number of islet cells also decreased, and a small amount of inflammatory cell infiltration also appeared in the islet. S1 drug is less effective than S9 drug, but shows relative good effects compared with model control group. S2 drug administration group was at the same level as the model control group, indicating that S2 drug did not have the function of protecting or repairing islets. Drugs S3-S7 are similar to drug S2 and do not show obvious efficacy; no detailed description will be provided herein.

These results show that S8 drug can not only significantly improve blood glucose index, but also protect islets from damage or repair islet damage, and its effects are much better than that of gold clusters crude product S9 and other drugs.

The above results indicate that the molecular structures of different gold cluster molecules containing the same ligand have great influence on the prevention and treatment of type 2 diabetes. For different gold cluster molecules containing ligand L-NIBC, gold cluster molecule $Au1_{18}(L\text{-}NIBC)_{14}$ (S8 drug) showed excellent effects on the prevention and treatment of type 2 diabetes, and has a very significant advantage in the prevention and treatment of type 2 diabetes over gold clusters crude product and other 7 gold cluster molecules.

On the other hand, islet injury is one of the typical characteristics of Type I diabetes. The excellent role of S8 drug in islet protection and islet repair is of great advantages and wide application prospects in the prevention and treatment of Type I diabetes.

Further research shows that gold clusters crude product S9 drug was added with S8 drug to obtain mixture drugs, and the therapeutic effects of the mixture drugs were significantly increased when the content of $Au_{18}(L\text{-}NIBC)_{14}$ in the

mixture drugs reached 7%.

**Embodiment 7. Parkinson's disease (PD) animal model experiments**

**7.1. Methods**

MPTP (1-methyl-4-phenyl-1,2,3,6-tetrahydropyridine) injury model is a widely used animal model in PD research. This study used this animal model. The experimental animals were SPF male C57BL/6 mice. The mice were randomly divided into blank control group, model control group, S1, S2, S3, S4, S5, S6, S7, S8 and S9 drug administration groups, with 12 mice in each group. Model control group mice were intraperitoneally injected of MPTP (30 mg/kg/day) for 7 days to establish the MPTP-induced PD mouse model, and blank control group was intraperitoneally injected of the same amount of normal saline. Each drug administration group was first intraperitoneally injected of MPTP (30 mg/kg/day), and 0.5 hour later, then intraperitoneally injected of corresponding drugs (10mg/kg/day), once a day for 7 consecutive days.

The effects of drugs on the behavior of model animals were analyzed by open field, rotarod test and swimming test. The specific methods are as follows:

Open field: for experiment, transfer the animals from a feeding cage to a spontaneous activity detector. After the animals adapt to new environment for 5min, start recording their spontaneous activities and changes within 5min, and then measure the strength of their action ability by the traveling distance and moving speed of the animals within 5min.

Rotarod test: animals need to keep balance and move continuously on a rotating rod. It is a widely used experiment to detect motion coordination. For experiment, the animals are placed on the rotating rod and avoid slipping. The diameter of the roller was 6cm and the rotating speed was 20 rpm/min. After five times of adaptation, the animals are tested for 15min each time, and the average value is taken for three consecutive times. When an animal slides down, the lower sensing platform will be stopped accordingly, and the latency of falling from the rotating rod will be recorded. The latency of falling from the rod and the number of animals falling from the rod will be recorded in each test.

Swim test: put to-be-tested mice into Morris water tank with a water depth of 60cm and a water temperature of 22°C. Record the swimming distance and swimming time of the animals within 10min to measure the strength of their action ability.

After the behavioral tests, the proteins in intracerebral striatum were detected by Western blot (WB). The required parts of the brain were removed from the ice, lysed with RIPA lysis buffer. Homogenate was centrifuged at 4°C, 12000g, 30 min, the protein was extracted, and the samples were prepared. SDS-polyacrylamide gel electrophoresis was conducted at 55V-60V for 4.5 hours, and proteins were transferred to membrane by a semi-dry method at 60 mA for about 1.5h. The membrane was blocked by 5% skimmed milk powder at room temperature for 1h; rabbit anti-tyrosine hydroxylase (TH) diluted with TBST (dilution ratio 1:300) was added and incubated overnight at 4°C. The antibody was recovered, and the membrane was washed with TBST for 3 times, 10 min/time. Add IRDye R 680RD Goat anti-rabbit diluted with TBST (dilution ratio 1:3000); washed with TBST 3 times, 10min/time. Protein signals were scanned by two color infrared laser imaging system.

All data are processed by Prism 5.0 software, and the data is expressed with $\bar{x}\pm$SD, using t-test or one-way ANOVA, $P < 0.05$ indicates that the difference is statistically significant.

**7.2. Results**

Fig. 17 shows the results of the behavioral tests.

The mice in model control group showed tremor, decreased movement, arched back, hind limb opening, unstable gait, vertical tail, and vertical hair at 3-5 min after MPTP administration, and one or two mice showed epileptic seizures. The above symptoms gradually decreased after 30-60 min, and basically returned to normal after 24h. However, with the increase of administration times, the acute reaction decreased, but after 24 hours, the decreased movement, gait instability and slow response became more and more obvious.

As shown in Figs. 17A and 17B, after injection of MPTP for 7 consecutive days, compared with blank control group, the spontaneous motion distance of mice in the model control group decreased by about 32% ($P < 0.01$, ##) and the moving speed decreased by about 29% ($P < 0.01$, ##), showing the symptoms of motion retardation. The spontaneous motion distance and moving speed of S8 drug administration group was significantly higher than that of model control group ($P < 0.01$, **), nearly recovered to level of blank control group. The spontaneous motion distance and moving speed of S9 drug administration group was also significantly higher than that of model control group ($P < 0.05$, *), but the increase amplitude is significantly less than that of S8 drug administration group (where the increase amplitude of spontaneous motion distance and moving speed of S8 drug administration group is 51% and 139% higher than that of S9 drug administration group, $P <0.05$). S1 drug administration group is similar to S9 drug administration group, showed significant increase compared with model control group ($P <0.05$), but the increase amplitude is significantly less than that of S8 drug administration group. S2-S7 drug administration groups showed no significant difference compared with model control group in terms of spontaneous motion distance and moving speed ($P > 0.05$).

In the swimming ability test of mice, the longer the swimming duration and the farther the swimming distance, the better the limb motor coordination. As shown in Figs. 17C and 17D, compared with the blank control group, the 10 minute-swimming distance of mice in the model control group decreased by about 27%, and the swimming duration decreased by about 18% ($P < 0.01$, ##). Compared with model control group, the swimming distance and swimming duration of S8 drug administration group increased significantly ($P < 0.01$, **), where the swimming distance increased 33%, and the swimming duration increased 21%. Compared with model control group, the swimming distance and swimming duration of gold clusters crude product S9 drug administration group also increased significantly (increased 21% and 11% respectively), but the increase amplitude is significantly smaller than that of S8 drug administration group (compared of S9 drug administration group with S8 drug administration group, $P < 0.05$, \$). S1 drug administration group was similar to S9 drug administration group, and also showed significant increase compared with model control group, but the increase amplitude is significantly smaller than that of S8 drug administration group. The values of remaining S2-S7 drugs were similar to those of model control group, and there was no significant difference from model control group ($P > 0.05$).

As shown in Figs. 17E and 17F, in the rotarod behavior test, mice in blank control group showed fall latency of 13.1 ± 2.1 min and drop rate of 31 ± 1.6%. The fall latency of mice in model control group was significantly shortened to 5.6 ± 1.8 min, and the fall rate was significantly increased to 78.1 ± 5.0%, indicating that MPTP modeling led to the decline of motor coordination ability, unstable grasping and easy to fall. Compared with model control group, the fall latency of S8 drug administration group increased significantly ($P < 0.01$, **), and the drop rate decreased extremely significantly ($P < 0.0001$, ***); these results demonstrated that S8 has the function of excellently improving MPTP-induced motor coordination function disorder. For gold clusters crude product S9 drug administration group, the fall latency was prolonged ($P < 0.05$, *), and the drop rate was also significantly deceased ($P < 0.01$, **), but the amplitude of increase and decrease was smaller than that of S8 drug administration group (comparing S8 with S9, significant difference in drop rate, $P < 0.05$, \$). S1 drug administration group was similar with S9 drug administration group, and also showed significant improvement of fall latency and drop rate compared with model control group ($P < 0.05$, *), but the amplitude of improvement is also evidently smaller than that of S8 drug administration group. The other drug administration groups were at the same or similar level with model control group in their fall latency and drop rate, and there was no significant difference ($P > 0.05$), indicating that there was no significant efficacy.

Western blot (WB) was used to further detect the expression of tyrosine hydroxylase (TH) protein in the intracerebral striatum. TH is the key enzyme of dopamine (DA) biosynthesis pathway. TH immunoblotting can show the expression level of TH in tissues, thus showing the changes of DA neurons. Fig. 18 shows the results of WB test. As shown in Fig. 18, MPTP treatment caused the TH expression level in model control group to decrease to 47.5 ± 3.7% of that in blank control group. The treatment of gold clusters crude product S9 caused the TH expression level in modeled mice to restore to 71.1 ± 2.3% of that in blank control group, showing significant difference in comparison with model control group ($P < 0.01$, **), showing that gold clusters crude product S9 has relative good protection against specific loss of DA neurons. The treatment of S8 drug caused the TH expression level in modeled mice to restore to 91.8 ± 3.3% of that in blank control group, showing extremely significant difference in comparison with model control group ($P$

< 0.001, ***), and also showing significant difference in comparison with S9 drug administration group ($P < 0.05$, \$), demonstrating that S8 drug has excellent protection against specific loss of DA neurons, and protection effect is superior to gold clusters crude product S9. The treatment of S1 drug also caused the TH expression level in modeled mice to restore to 60.7 ± 4.2% ($P < 0.05$, *), but the restoration amplitude is apparently less than that of S9 administration group, and even less than that of S8 drug administration group. The TH expression amounts in S2-S7 drug administration groups were at the same level as that of model control group, indicating that S2-S7 drugs have no significant neuroprotective effect.

The above results indicate that the molecular structures of different gold cluster molecules containing the same ligand have great influence on their prevention and treatment of PD. For different gold cluster molecules containing ligand L-NIBC, gold cluster molecule $Au_{18}(L-NIBC)_{14}$ (S8 drug) showed excellent therapeutic effect on AD treatment. gold cluster molecule $Au_{25}(L-NIBC)_{18}$ (S1 drug) and gold clusters crude product S9 drug also have evident therapeutic effect, but the effects from either behavior test or TH WB are less than that of gold cluster molecule $Au_{18}(L-NIBC)_{14}$ (S8 drug). The other 6 gold cluster molecules had no obvious therapeutic effect. This indicates that, compared with other 7 gold cluster molecules and gold clusters crude product S9, gold cluster molecule $Au_{18}(L-NIBC)_{14}$ has a very significant advantage in the prevention and treatment of PD, more suitable for manufacturing drugs for prevention and treatment of PD.

Further research shows that gold clusters crude product S9 drug was added with S8 drug to obtain mixture drugs, and the therapeutic effects of the mixture drugs were significantly increased when the content of $Au_{18}(L-NIBC)_{14}$ in the mixture drugs reached 7%.

**Embodiment 8. Ischemia reperfusion glaucoma animal model**

## 8.1. Methods

100 male SD rats, according to the b-wave amplitude of ERG before modeling, were randomly divided into 10 groups. The right eye was subjected to eye ischemia-reperfusion modeling, the left eye was not subjected to modeling treatment (as blank control), and the administration began on the day of modeling (counted as the first day, D1). Except that model control group was not treated, the rest were all drug administration groups, including S1, S2, S3, S4, S5, S6, S7, S8 and S9 drug administration groups, which were administered by eye drops at the dose of 1mg/eye, three times a day for 21 consecutive days. The drug administration time of D1 was set to about 3h, 2h and 1h before modeling, and the drug administration interval of the rest administration days except for D1 was 3h. During the test, the animals were observed in general clinical observation and local eye observation every day, and their body weight was measured once a week. Before drug administration and on 7th (D8), 14th (D15) and 21st (D22) days after the first drug administration, the animals were examined by full field electroretinogram (ERG) and optical coherence tomography (OCT).

After the last examination, all animals were euthanized for gross anatomical observation, and the eyeballs of both eyes were collected and fixed in Davidson's solution for routine histological treatment, including sampling, paraffin embedding, sectioning and HE staining. After removing the surrounding connective tissue, the optic nerve was quickly placed in precooled PFA fixation solution, repaired quickly, dehydrated, resin embedded, ultra-thin section and toluidine blue staining. The structures of various parts of the eyeball and optic nerve were observed under light microscope, focusing on the morphology of optic nerve axons, the thickness of each layer of retina and the morphological changes of cells. The pathologist examined and evaluated the sections with a light microscope. The lesions were diagnosed and graded using standard terminology. The microscopic results were graded and scored by 4 grading method, which were mild (1 point), minor (2 points), moderate (3 points) and severe (4 points) respectively, so as to facilitate the comparison between groups.

Statistical analysis of data: the data were processed by statistical software SPSS 22.0. First, Levene test was used to test the homogeneity of the data. If the data were uniform ($P > 0.05$), one-way ANOVA was performed; if Levene test analysis shows that the data is non normal distribution ($P \leq 0.05$), Kruskal Wallis nonparametric test shall be performed. If the result of Kruskal Wallis nonparametric test is significant ($P \leq 0.05$), Mann Whitney U test is further used for pairwise comparison. $P < 0.05$ was considered as statistically significant.

## 8.2. Results

Electroretinogram (ERG) is a common method to evaluate the optic nerve function damages of ischemia-reperfusion glaucoma animals. A group of complex potential changes generated by retinal visual sensory cells under light stimulation can be led out by electrodes and recorded by an appropriate magnifying device, that is, ERG diagram, which directly reflects the changes of retinal optic nerve function.

Fig. 19A shows exemplary ERG (DA3.0) in the left eye (normal eye) (right panel) and right eye (modeling eye) (left panel) of rats in model control group on 7th day (D8) after modeling. As shown in Fig. 19A, there is no significant difference in the amplitude of a wave between left and right eyes, but there is a significant change in b wave. As a blank control, b wave of normal left eye was clearly visible with normal amplitude, but the amplitude of b wave of modeling right eye decreased significantly and the waveform became unclear. The b-wave amplitude of DA10.0 in the right and left eyes was similar to that of DA3.0, and also changed significantly. The above results showed that the optic nerve function of modeling eye decreased severely and the modeling was successful.

Figs. 19B and 19C show the results of b-wave amplitude of full field visual ERG DA3.0 and DA10.0 in the right eye of rats in each group respectively. During modeling and prior to drug administration, there was no significant difference in the amplitudes of b wave in the right eye among rats in each group. Compared with model control group in the same period, the b-wave amplitudes of DA3.0 (Fig. 19B) in the right eye of rats in S8 drug administration group were significantly higher than those in model control group at D8, D15 and D22 (D8: $P < 0.01$, **; D15: $P < 0.05$, *; D22: $P < 0.05$, *), and the b-wave amplitudes of DA10.0 (Fig. 19C) were also significantly higher than that of model control group (at all days, $P < 0.05$, *).

Fig. 19D shows exemplary ERG DA3.0 in the left eye (normal eye) (right panel) and the right eye (modeling and drug administration eye) (left panel) of rats in S8 drug administration group on 7th day after administration (D8). It can be seen that ERG waveform of the right eye is clear and normal after S8 drug administration, and there is no significant difference in b-wave shape and amplitude between the right eye and the left eye. The b-wave amplitudes of ERG DA10.0 in the right and left eyes were similar to that of DA3.0, and there is no significant difference between the left eye and the right eye, so it will not be described in detail herein. These results show that S8 drug can significantly improve the damage of optic nerve function in glaucoma rat model induced by ischemia-reperfusion.

The amplitude of b wave DA3.0 in the right eye of rats in S9 drug administration group was also higher than that in the right eye of rats in model control group, and showed significant difference at D15 and D22 ($P < 0.05$, *), but the increase amplitude was evidently lower than that in the right eye of rats in S8 drug administration group (where there is significant difference at D8, $P < 0.05$, $). The amplitude of b wave DA10.0 in the right eye of rats in S9 drug administration group was also higher than that in the right eye of rats in model control group, and showed significant difference at D8 ($P < 0.05$, *). These results showed that gold clusters crude product S9 has effects of improving the damage of optic nerve function in glaucoma rat model induced by ischemia-reperfusion, but the effects of improvement are apparently less than that of S8 drug.

The amplitudes of b wave DA3.0 in the right eye of rats in S1 drug administration group were significantly higher than those in the right eye of rats in model control group at D8 ($P < 0.05$, *); while there were increases to certain extent at D15 and D22, the difference was not significant. The amplitudes of b wave DA10.0 in the right eye of rats in S1 drug administration group were significantly higher than those in the right eye of rats in model control group. These results show that S1 drug has effects of improving the damage of optic nerve function in glaucoma rat model induced by ischemia-reperfusion, but the effects of improvement are apparently less than that of S8 drug.

S2-S7 drugs did not show any therapeutic effect.

Optical coherence tomography (OCT) was further used to examine the thickness changes of rat retina. The results showed that the retinal thickness of the right eye in model control group at 750μm or 1500μm from optic disc showed a gradual downward trend with the increase of days. At 1500μm from optic disc, for example, the retinal thickness decreased by about 11% at 21st day (D22) after modeling compared with that before modeling, and the difference was significant ($P < 0.01$). After modeling and drug administration, the thickness of the right eye of rats in S8 drug administration group was significantly higher than that of rats in model control group on the corresponding days, and the differences were significant at 14th day (D15) ($P < 0.05$) and at 21st day (D22) ($P < 0.01$). This result also shows the protective effect of S8 drug on rat retina.

Fig. 20 shows the pathological changes of fundus induced by ischemia-reperfusion modeling and drug administration by staining ultrathin sections by toluidine blue. Fig. 20A shows the results of pathological scores; Figs. 20B, 20C and 20D show the exemplary images of the left eye as blank control, the right eye in model control group, and the right eye in S8 drug administration group respectively. The pathological examination showed that the optic nerve structure of the left eye as blank control was clear, the myelin sheath was with rings of different sizes, and no obvious abnormality was found (Fig. 20B). Severe optic nerve structure disorder and myelin loss were observed in the right eye of model control group after modeling (Fig. 20C). Compared with the right eye of model control group, the right eye of the rats in S9 administration drug group showed better improvement effect, but the optic nerve structure was disordered to a certain extent, myelin sheath loss was obvious, and the pathological score was significantly lower than that of the right eye of model control group (Fig. 20A) ($P < 0.05$, *). Compared with the right eye of model control group, the right eye of S2 drug administration group showed an extremely obvious improvement, the optic nerve structure was clear, similar to the normal left eye, only a small amount of slight demyelination was observed (Fig. 20D), and the pathological score was significantly lower than that of the right eye of model control group (Fig. 20A) ($P < 0.001$, ***). Compared with the right eye of rats in S9 drug administration group, the pathological score of the right eye of rats in the S8 drug administration group is significantly lower ($P < 0.05$, $), indicating that the therapeutic effect of S8 drug is much better than that of gold clusters crude product S9 drug. S1 drug was similar to S9 drug, also showing certain effects of improvement, but significantly less than that of S8 drug, which will not be described in detail herein. S2-S7 drugs showed no apparent effect.

The above results show that the molecular structures of different gold cluster molecules containing the same ligand have a great impact on their effects in optic nerve protection and the prevention and treatment of glaucoma. For different gold cluster molecules containing L-NIBC, gold cluster molecule $Au_{18}(L\text{-}NIBC)_{14}$ (S8 drug) has extremely excellent therapeutic effect; gold clusters crude product S9 drug and gold cluster molecule $Au_{25}(L\text{-}NIBC)_{18}$ (S1 drug) also have evident therapeutic effect, but much less than gold cluster molecule $Au_{18}(L\text{-}NIBC)_{14}$. The other 6 gold cluster molecules has not apparent therapeutic effect. It can be seen from the above results that gold cluster molecule $Au_{18}(L\text{-}NIBC)_{14}$ has extremely significant advantage in the prevention and cure of glaucoma.

Further research shows that gold clusters crude product S9 drug was added with S8 drug to obtain mixture drugs, and the therapeutic effects of the mixture drugs were significantly increased when the content of $Au_{18}(L\text{-}NIBC)_{14}$ in the mixture drugs reached 7%.

**Claims**

1. An L-N-isobutyryl cysteine (L-NIBC)-bound gold cluster molecule comprising:

    a gold core; and
    a ligand L-NIBC bound to the gold core;

    wherein the L-NIBC-bound gold cluster molecule consisting of 18 gold atoms, and 14 L-NIBC ligands which has a formula of $Au_{18}(L\text{-}NIBC)_{14}$.

2. A composition comprising the L-NIBC-bound gold cluster molecule $Au_{18}(L\text{-}NIBC)_{14}$ of claim 1, wherein the L-NIBC-bound gold cluster molecule $Au_{18}(L\text{-}NIBC)_{14}$ is used as a component in a mixture of gold cluster molecules in the composition, the L-NIBC-bound gold cluster molecule $Au_{18}(L\text{-}NIBC)_{14}$ has a chromatographic content of at least 7% in the mixture.

3. The L-NIBC-bound gold cluster molecule $Au_{18}(L\text{-}NIBC)_{14}$ of claim 1 or the composition of claim 2 for use as a medicament.

4. The L-NIBC-bound gold cluster molecule $Au_{18}(L\text{-}NIBC)_{14}$ of claim 1 or the composition of claim 2 for use in treatment of multiple sclerosis.

5. The L-NIBC-bound gold cluster molecule $Au_{18}(L\text{-}NIBC)_{14}$ of claim 1 or the composition of claim 2 for use in treatment of Alzheimer's disease (AD).

6. The L-NIBC-bound gold cluster molecule $Au_{18}(L\text{-}NIBC)_{14}$ of claim 1 or the composition of claim 2 for use in treatment of liver cirrhosis.

7. The L-NIBC-bound gold cluster molecule $Au_{18}(L\text{-}NIBC)_{14}$ of claim 1 or the composition of claim 2 for use in treatment of diabetes.

8. The L-NIBC-bound gold cluster molecule $Au_{18}(L\text{-}NIBC)_{14}$ of claim 1 or the composition of claim 2 for use in treatment of Parkinson's disease (PD).

9. The L-NIBC-bound gold cluster molecule $Au_{18}(L\text{-}NIBC)_{14}$ of claim 1 or the composition of claim 2 for use in treatment of glaucoma.

**Patentansprüche**

1. Ein L-N-Isobutyrylcystein (L-NIBC)-gebundenes Goldcluster-Molekül, umfassend:

   einen Goldkern; und
   einen an den Goldkern gebundenen Liganden L-NIBC;

   wobei das an L-NIBC gebundene Goldclustermolekül aus 18 Goldatomen und 14 L-NIBC-Liganden besteht und die Formel $Au_{18}(L\text{-}NIBC)_{14}$ aufweist.

2. Eine Zusammensetzung, die das L-NIBC-gebundene Goldclustermolekül $Au_{18}(L\text{-}NIBC)_{14}$ nach Anspruch 1, wobei das L-NIBC-gebundene Goldclustermolekül $Au_{18}(L\text{-}NIBC)_{14}$ als Bestandteil in einer Mischung aus Goldclustermolekülen in der Zusammensetzung verwendet wird und das L-NIBC-gebundene Goldclustermolekül $Au_{18}(L\text{-}NIBC)_{14}$ einen chromatographischen Gehalt von mindestens 7 % in der Mischung aufweist.

3. Das L-NIBC-gebundene Goldclustermolekül $Au_{18}(L\text{-}NIBC)_{14}$ nach Anspruch 1 oder die Zusammensetzung nach Anspruch 2 zur Verwendung als Arzneimittel.

4. Das L-NIBC-gebundene Goldclustermolekül $Au_{18}(L\text{-}NIBC)_{14}$ nach Anspruch 1 oder die Zusammensetzung nach Anspruch 2 zur Verwendung bei der Behandlung von Multipler Sklerose.

5. Das L-NIBC-gebundene Goldclustermolekül $Au_{18}(L\text{-}NIBC)_{14}$ nach Anspruch 1 oder die Zusammensetzung nach Anspruch 2 zur Verwendung bei der Behandlung der Alzheimer-Krankheit (AD).

6. Das L-NIBC-gebundene Goldclustermolekül $Au_{18}(L\text{-}NIBC)_{14}$ nach Anspruch 1 oder die Zusammensetzung nach Anspruch 2 zur Verwendung bei der Behandlung von Leberzirrhose.

7. Das L-NIBC-gebundene Goldclustermolekül $Au_{18}(L\text{-}NIBC)_{14}$ nach Anspruch 1 oder die Zusammensetzung nach Anspruch 2 zur Verwendung bei der Behandlung von Diabetes.

8. Das L-NIBC-gebundene Goldclustermolekül $Au_{18}(L\text{-}NIBC)_{14}$ nach Anspruch 1 oder die Zusammensetzung nach Anspruch 2 zur Verwendung bei der Behandlung der Parkinson-Krankheit (PD).

9. Das L-NIBC-gebundene Goldclustermolekül $Au_{18}(L\text{-}NIBC)_{14}$ nach Anspruch 1 oder die Zusammensetzung nach Anspruch 2 zur Verwendung bei der Behandlung von Glaukom.

**Revendications**

1. Molécule d'agrégat d'or liée à de la L-*N*-isobutyryl cystéine (L-NIBC), comprenant :

    un noyau d'or ; et
    un ligand de L-NIBC lié au noyau d'or ;
    dans laquelle la molécule d'agrégat d'or liée à de la L-NIBC est constituée de 18 atomes d'or, et de 14 ligands de L-NIBC présentant une formule de $Au_{18}(L\text{-}NIBC)_{14}$.

2. Composition comprenant la molécule d'agrégat d'or liée à de la L-NIBC $Au_{18}(L\text{-}NIBC)_{14}$ selon la revendication 1, dans laquelle la molécule d'agrégat d'or liée à de la L-NIBC $Au_{18}(L\text{-}NIBC)_{14}$ est utilisée comme composant dans un mélange de molécules d'agrégat d'or dans la composition, la molécule d'agrégat d'or liée à de la L-NIBC $Au_{18}(L\text{-}NIBC)_{14}$ présentant une teneur chromatographique d'au moins 7 % dans le mélange.

3. Molécule d'agrégat d'or liée à de la L-NIBC $Au_{18}(L\text{-}NIBC)_{14}$ selon la revendication **1** ou composition selon la revendication 2, pour utilisation comme médicament.

4. Molécule d'agrégat d'or liée à de la L-NIBC $Au_{18}(L\text{-}NIBC)_{14}$ selon la revendication **1** ou composition selon la revendication 2 pour utilisation dans le traitement de la sclérose en plaques.

5. Molécule d'agrégat d'or liée à de la L-NIBC $Au_{18}(L\text{-}NIBC)_{14}$ selon la revendication **1** ou composition selon la revendication 2, pour utilisation dans le traitement de la maladie d'Alzheimer (MA).

6. Molécule d'agrégat d'or liée à de la L-NIBC $Au_{18}(L\text{-}NIBC)_{14}$ selon la revendication **1** ou composition selon la revendication 2, pour utilisation dans le traitement de la cirrhose du foie.

7. Molécule d'agrégat d'or liée à de la L-NIBC $Au_{18}(L\text{-}NIBC)_{14}$ selon la revendication **1** ou composition selon la revendication 2 pour utilisation dans le traitement du diabète.

8. Molécule d'agrégat d'or liée à de la L-NIBC $Au_{18}(L\text{-}NIBC)_{14}$ selon la revendication **1** ou composition selon la revendication 2, pour utilisation dans le traitement de la maladie de Parkinson (PD).

9. Molécule d'agrégat d'or liée à de la L-NIBC $Au_{18}(L\text{-}NIBC)_{14}$ selon la revendication **1** ou composition selon la revendication 2 pour utilisation dans le traitement du glaucome.

Fig. 1A

Fig. 1B

[Au₁₈(L-NIBC)₁₄-5H]⁵⁻

Fig. 1C

[Au₁₈(L-NIBC)₁₄-4H]⁴⁻

Fig. 1D

[Au₁₈(L-NIBC)₁₄-3H]³⁻

Fig. 1E

[Au₁₈(L-NIBC)₁₄-2H]²⁻

Fig. 1F

Fig. 1

**Fig. 2**

**Fig. 3**

**Fig. 4**

**Fig. 5**

**Fig. 6A**

**Fig. 6B**

**Fig. 6C**

**Fig. 6D**

**Fig. 6E**

**Fig. 6F**

**Fig. 6**

**Fig. 7**

Fig. 8A

Fig. 8B

**Fig. 8C**

**Fig. 8D**

**Fig. 8**

**Fig. 9**

**Fig. 10A**

**Fig. 10B**

**Fig. 10C**

**Fig. 10D**

**Fig. 10E**

**Fig. 10F**

**Fig. 10**

Fig. 11A

Fig. 11B

Fig. 11C

Fig. 11D

Fig. 11

Fig. 12A

Fig. 12B

Fig. 12C

Fig. 12D

Fig. 12E

Fig. 12

Fig. 13A

Fig. 13B

Fig. 13C

Fig. 13D

Fig. 13E

Fig. 13F

**Fig. 13**

**Fig. 14A**

Time points after intragastric
administration of glucose (h)

**Fig. 14B**

**Fig. 14**

Fig. 15A

Fig. 15B

Fig. 15C

Fig. 15D

Fig. 15

Fig. 16A

Fig. 16B

Fig. 16C

Fig. 16D

Fig. 16E

Fig. 16F

Fig. 16G

**Fig. 16**

**Fig. 17A**

Fig. 17B

Fig. 17C

Fig. 17D

Fig. 17E

Fig. 17F

Fig. 17

Fig. 18

**Fig. 19A**

**Fig. 19B**

Fig. 19C

Fig. 19D

Fig. 19

Fig. 20A

Fig. 20B

Fig. 20C

Fig. 20D

Fig. 20

**REFERENCES CITED IN THE DESCRIPTION**

**Patent documents cited in the description**

- WO 2021184762 A1

- WO 2021226736 A1